(19) Europäisches Patentamt / European Patent Office / Office européen des brevets

(11) **EP 3 278 818 B1**

(12) **EUROPEAN PATENT SPECIFICATION**

(45) Date of publication and mention
of the grant of the patent:
**29.09.2021 Bulletin 2021/39**

(51) Int Cl.:
*A61L 27/12* (2006.01)       *C01B 25/32* (2006.01)
*A61L 27/50* (2006.01)       *B01J 27/18* (2006.01)
*H01M 10/0562* (2010.01)

(21) Application number: **16382381.8**

(22) Date of filing: **02.08.2016**

(54) **PERMANENTLY POLARIZED HYDROXYAPATITE, A PROCESS FOR ITS MANUFACTURE AND USES THEREOF**

PERMANENT POLARISIERTES HYDROXYAPATIT, VERFAHREN ZU DESSEN HERSTELLUNG UND DESSEN VERWENDUNG

HYDROXYAPATITE POLARISÉE DE MANIÈRE PERMANENTE, SON PROCÉDÉ DE FABRICATION ET SES UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**07.02.2018 Bulletin 2018/06**

(73) Proprietors:
• **B. Braun Surgical, S. A.**
**08191 Rubi (ES)**
• **Universitat Politècnica De Catalunya**
**08034 Barcelona (ES)**

(72) Inventors:
• **TURON DOLS, Pau**
**08191 Rubí (ES)**
• **del VALLE MENDOZA, Luis Javier**
**08034 Barcelona (ES)**
• **PUIGGALÍ BELLALTA, Jordi**
**08034 Barcelona (ES)**
• **ALEMÁN LLANSÓ, Carlos Enrique**
**08034 Barcelona (ES)**

(74) Representative: **Ponti & Partners, S.L.P**
**C. de Consell de Cent 322**
**08007 Barcelona (ES)**

(56) References cited:
• **UESHIMA M ET AL: "Electrovectorial effect of polarized hydroxyapatite on quasiepitaxial growth at nano-interfaces", SOLID STATE IONICS, NORTH HOLLAND PUB. COMPANY. AMSTERDAM; NL, NL, vol. 151, no. 1-4, 1 November 2002 (2002-11-01), pages 29-34, XP004393763, ISSN: 0167-2738, DOI: 10.1016/S0167-2738(02)00600-8**
• **MIHO NAKAMURA ET AL: "Role of blood coagulation components as intermediators of high osteoconductivity of electrically polarized hydroxyapatite", JOURNAL OF BIOMEDICAL MATERIALS RESEARCH. PART A, vol. 79A, no. 3, 1 December 2006 (2006-12-01), pages 627-634, XP055341307, HOBOKEN, NY, US ISSN: 1549-3296, DOI: 10.1002/jbm.a.30827**
• **GITTINGS ET AL: "Characterisation of ferroelectric-calcium phosphate composites and ceramics", JOURNAL OF THE EUROPEAN CERAMIC SOCIETY, ELSEVIER SCIENCE PUBLISHERS, BARKING, ESSEX, GB, vol. 27, no. 13-15, 1 January 2007 (2007-01-01), pages 4187-4190, XP022143720, ISSN: 0955-2219, DOI: 10.1016/J.JEURCERAMSOC.2007.02.120**
• **Tofail, S.A.M., Bauer J.: "Electro-thermal Polarisation of Hydroxyapatite Ceramics and Coatings for Bone Tissue Engineering Applications" In: Tofail, S.A.M, Bauer, J.: "Electrically Active Materials for Medical Devices", 2016, Imperial College Press, London, GB, XP8183113, ISBN: 9781783269860 pages 115-134, * 1. Electro-thermally Polarised Hydroxyapatite (HA) Ceramics ***

EP 3 278 818 B1

EP 3 278 818 B1

- YU ET AL: "Local structure of hydroxy-peroxy apatite: A combined XRD, FT-IR, Raman, SEM, and solid-state NMR study", JOURNAL OF PHYSICS AND CHEMISTRY OF SOLIDS, PERGAMON PRESS, LONDON, GB, vol. 68, no. 10, 1 October 2007 (2007-10-01), pages 1863-1871, XP022283261, ISSN: 0022-3697, DOI: 10.1016/J.JPCS.2007.05.020

**Description**

**Field of the invention**

The present invention relates to a a process for manufacturing a permanently polarized hydroxyapatite.

**Background of the invention**

Hydroxyapatite (HAp), $Ca_{10}(PO_4)_6(OH)_2$, is the major inorganic component of biological hard tissues such as bone and tooth.[1,2] Synthetic HAp, which shows excellent ability to interact with living systems, has been investigated for biomedical applications, as for example drug and gene delivery, tissue engineering and bone repair.[3-8]

An important difference between amorphous calcium phosphate (ACP) and crystalline synthetic HAp (cHAp) is the alignment of the $OH^-$ ions along the c-axis in the latter. The crystal structure of stoichiometric cHAp, which contains no $OH^-$ defects, is monoclinic at room temperature.[9,10] The monoclinic cHAp changes to hexagonal phase at about 210°C, which means a change from an ordered to a disordered distribution of $OH^-$ ions along the c-axis. In addition to thermal phase transition, $OH^-$ defects also cause a phase transition.[9,10] In this case, the hexagonal phase becomes the most stable form of cHAp in the pH range of 4-12 because of the disorder caused by the presence of vacancies and presence of oxygen radicals in the columns of $OH^-$ groups. Although electrical and dielectric properties of cHAp were found to be altered by thermally-induced changes in the positions of $OH^-$ ions,[11-13] the observed polarization effects were not stable at room temperature (i.e. the $OH^-$ re-reorientation has a short relaxation time).

Yamashita and co-workers[14,15] provoked persistent polarization effects in the polycrystalline HAp samples by applying a constant DC electric field of 1.0-4.0 kV/cm to samples sintered previously at 1250 °C for 2 h. This approach is based on a constant electric field. Results indicated that the persistent polarization was consequence of the electrical dipoles associated to the formation of defects inside crystal grains and of the space charge polarization originated in the grain boundaries. The thermally stimulated polarization process was found to exert different effects on the HAp surface properties.[16,17] Although the influence of polarization exhibited no effect on the surface roughness, crystallinity and constituent elements of cHAp, the wettability[16] and adhesion of osteoblastic cells is higher onto polarized samples than onto as prepared ones.[17] The latter phenomena were attributed to the increase in the surface free energies in comparison with non-polarized cHAp surfaces.

In this sense, document ES2323628 discloses that calcium hydroxyapatite in solid solution is obtained by sintering the prepared powder by a given method at 1200°C for 1-5 hours. The ceramic material can be polarized at a T higher than 1000°C or at a voltage higher than 100.000 V/cm. Nevertheless, the energy is not stored in such conditions and accordingly it is better to work under 1000°C or a voltage between 10 and 100.000 V/cm.

Fu, Cong et al. discloses in "Hydroxyapatite thin films with giant electrical polarization", chemistry of Materials (2015) 27(4), 1164-1171, that films of hydroxyapatite formed on titanium and stainless steel electrodes are found to display giant polarization with quasipermanent stored charge in excess of 70.000 microcoulombs per square centimeter.

Recently, the present inventors examined the capacity of prepared ACP and cHAp to interact with different phosphates and a biophosphonate (BPs),[18] which is a very relevant topic in the field of biomaterials for biomedical applications. Thus, polyphosphate (polyP), which is an orthophosphate polymer found in mammalian organisms,[19] promotes bone regeneration when adsorbed onto HAp.[20-24] Specifically, polyP stabilizes basic cell growth and differentiation enhancing bone regeneration.[25-27] Further, other studies reported that polyP and pyrophosphate $\left( P_2O_7^{4-} \right)$ inhibit HAp crystal growth.[28,29] More recently, Grynpas and coworkers[30] proposed that the production of polyP plays an important role in cartilage mineralization and bone formation, which was attributed to the local accumulation of phosphate $\left( PO_4^{3-} \right)$ and calcium ($Ca^{2+}$) through the formation of strong complexes. This hypothesis was supported by both the adsorption of polyP onto HAp and the correlation between the hydrolytic correlation of polyP in $Ca^{2+}$-polyP complexes and the increment of $PO_4^{3-}$ and $Ca^{2+}$ concentrations. On the other hand, in BPs the oxygen atom that links the phosphate groups of pyrophosphates is replaced by a carbon atom, which results in the inhibition of both hydrolytic and enzymatic degradations.[31] The affinity of BPs towards HAp increases by incorporating amino functionalities to the tertiary carbon atom, which has been associated to the formation of strong hydrogen bonds between the two species.[32,33] Furthermore, BPs are primary agents in the current pharmacological arsenal against different bone diseases (e.g. osteoporosis, Paget disease of bone and malignancies metastatic to bone).[34]

Recent observations evidenced that the adsorption of polyP and $P_2O_7^{4-}$ onto as prepared ACP and cHAp is favored at pH 7 with respect to basic pH 9, even though some limitations in the association processes were found when the results obtained using different adsorbate concentrations were compared.[18] Studies on the adsorption of aminotris(methylenephosphonic acid), hereafter denoted ATMP, suggested that the affinity of ACP and cHAp towards this BP is lower than towards polyP and $P_2O_7^{4-}$ .[18]

Ueshima M et al. disclose in "Electrovectorial effect of polarized hydroxyapatite on quasiepitaxial growth at nano-interfaces", Solid State Ionics, North Holland Pub. Company Amsterdam; Vol. 151 (1-4), 1 Nov. 2002, pp. 29-34, a process for obtaining a polarized hydroxyapatite comprising the step of applying an electrical field of 10 kV/cm for 1 h, at a T of 800 °C.

Miho Nakamura et al. disclose in "Role of blood coagulation components as intermediators of high osteoconductivity of electrically polarized hydroxyapatite", Journal of biomedical Material Research, Part A, vol. 79 , no, 3, 1 December 2006, pp 627.634, the preparation of polarized hydroxyapatite samples by applying a d.c. field of 1,0 kV cm-1 with a pair of platinum electrodes in air at 300 °C for 1 hour.

Yu et al. disclose in "Local structure of hydroxy-peroxy apatite: A combined XRD, FT-IR, Raman, SEM, and solid-state NMR study", Journal of Physics and Chemistry of Solids, Pergamon Press London, GB, Vol. 68, no. 10, 1 October 2007, pp. 1863-1871, the structure of synthesized hydroxyapatite and hydroxy-peroxy apatite using various techniques, such as X-ray powder diffraction, FT-IR Raman spectroscopy scanning electron microscopy and solid-state NMR spectroscopy.

Accordingly, in view of above, the present inventors have surprisingly found that it is possible to obtain a permanently polarized hydroxyapatite with specific electrochemical and electrical properties associated with a huge range of possibilities of use as disclosed below.

## Summary of the invention

A first aspect of the present invention relates to a process for obtaining a permanently polarized hydroxyapatite.

## Brief description of the drawings

Figure 1. FTIR spectra of (a) cHAp and (b) ACP.

Figure 2. X-ray diffraction patterns of the cHAp and ACP particles studied in this work: (a) cHAp/p and cHAp/tsp; and (b) ACP/p and ACP/tsp. cHAp and ACP samples were identified by the peaks at $2\theta=32°-34°$.

Figure 3. High-resolution XPS spectra for (a) cHAp/p, (b) cHAp/s and (c) cHAp/tsp samples: P2p, Ca2p, and O1s regions.

Figure 4. SEM micrographs of cHAp/p, c/HAp/s and cHAp/tsp particles.

Figure 5. For cHAp/p, c/HAp/s and cHAp/tsp: (a) control voltammograms and variation of both (b) the loss of electroactivity (LEA in Eqn 2) and (c) the specific capacitance (C in Eqn 3) with the number of consecutive oxidation-reduction cycles in PBS.

Figure 6. (a) Nyquist and (b) Bode plots for cHAp/p, cHAp/s and cHAp/tsp. (c) Electrical equivalent circuit (EEC) used to fit the experimental data recorded for cHAp/s and cHAp/tsp: Rs is the electrolyte resistance, $CPE_b$ and $R_b$ are the bulk constant phase element and resistance, respectively, $CPE_{dl}$ is the contribution of the double layer capacitance. Open symbols correspond to phase angle values whereas solid symbols correspond to Log|Z|, and black lines correspond to the fitted profile. Inset in figure a) represents the Nyquist behavior at high frequency.

Figure 7. Contact angle of the first and second FBS drops ($\theta_{FBS}$ and $\theta'_{FBS}$ in black and grey, respectively) for cHAp/s and cHAp/tsp samples before and after incubation in presence of $P_2O_7^{4-}$ , polyP and ATMP.

Figure 8. FTIR spectra of cHAp/p, cHAp/s and cHAp/tsp incubated in presence of (a) polyP (200 mM), (b) $P_2O_7^{4-}$ (100 mM) and (c) ATMP (200 mM) at pH 7. Arrows indicate the position of the bands and shoulder used to identify the adsorption of the different adsorbates.

Figure 9. For (a and c) cHAp/s and (b and d) cHAp/tsp: (a and b) control voltammograms and (c and d) variation of the loss of electroactivity (LEA in Eqn 2) with the number of consecutive oxidation-reduction cycles in PBS for samples non-incubated and incubated in presence of $P_2O_7^{4-}$ , polyP and ATMP.

Figure 10. X-ray diffraction patterns of the cHAp/p and cHAp/s samples, which were identified by the peaks at $2\theta=32°-34°$.

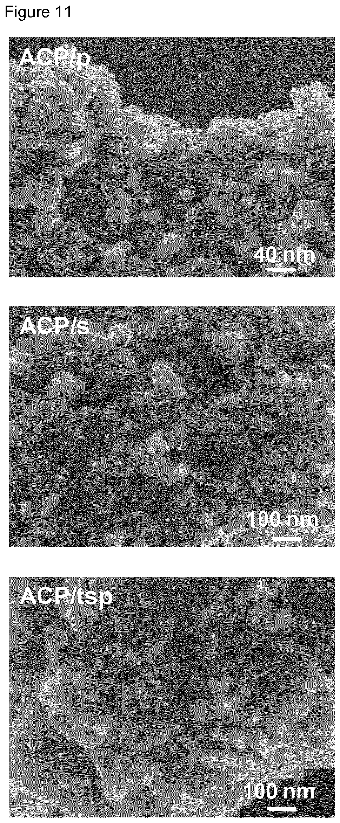

Figure 11. SEM micrographs of ACP/p, ACP/s and ACP/tsp particles. The nanospherical morphologies found in ACP/p transforms into fusiform nanorods in ACP/tsp, whereas the two morphologies seem to coexist in ACP/s.

**Figure 12.** High-resolution XPS spectrum in the Na1s region for (a) cHAp/s and (b) cHAp/tsp samples before and after incubation in presence of ATMP, $P_2O_7^{4-}$ and polyP.

**Figure 13.** High-resolution XPS spectrum in the N1s region for (a) cHAp/s and (b) cHAp/tsp samples before and after incubation in presence of ATMP.

**Figure 14.** For cHAp/p: (a) control voltammogram and (b) variation of the loss of electroactivity (LEA in Eqn 2) with the number of consecutive oxidation-reduction cycles in PBS for samples non-incubated and incubated in presence of $P_2O_7^{4-}$, polyP and ATMP.

**Figure 15.** a) Nyquist, (b) log |Z| and (c) phase angle plots for cHAp/s alone and incubated in presence of polyP (200 mM), $P_2O_7^{4-}$ (100 mM) and (c) ATMP (200 mM) at pH 7.

**Figure 16.** a) Nyquist, (b) log |Z| and (c) phase angle plots for cHAp/tsp alone and incubated in presence of polyP (200 mM), $P_2O_7^{4-}$ (100 mM) and (c) ATMP (200 mM) at pH 7.

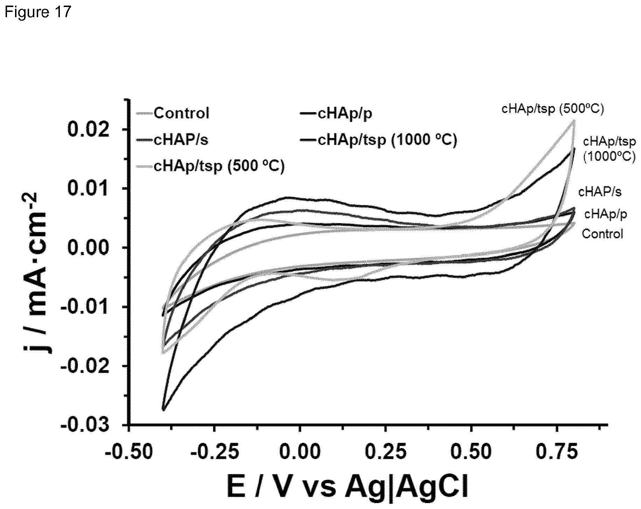

**Figure 17** Figure 17 shows the control voltammograms for cHAp/p, c/HAp/s and cHAp/tsp in PBS. The electrochemical activity, which is defined by the anodic and cathodic areas of the voltammogram, is noticeably higher for HAp/tsp than for HAp/s and HAp/p and the control (stainless steel, AISI 304, electrode).

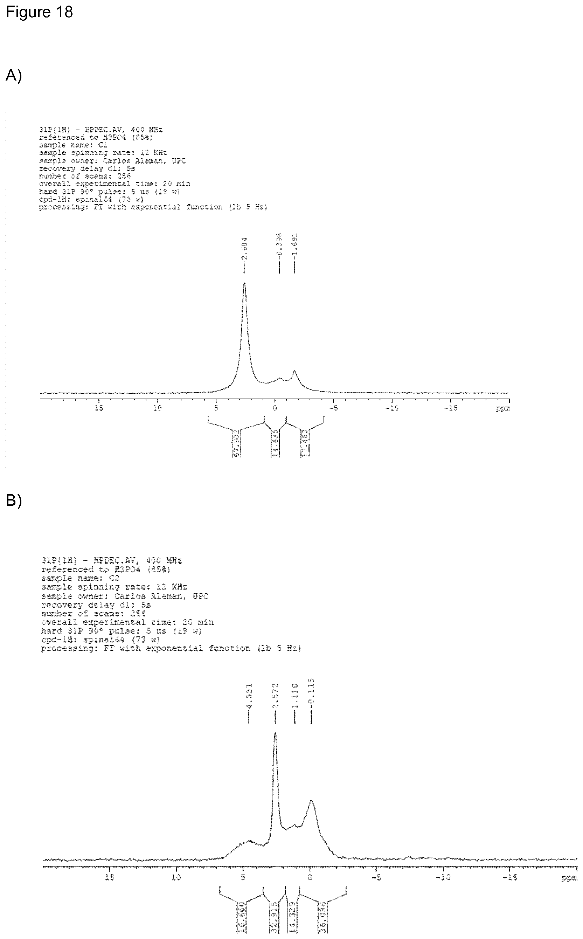

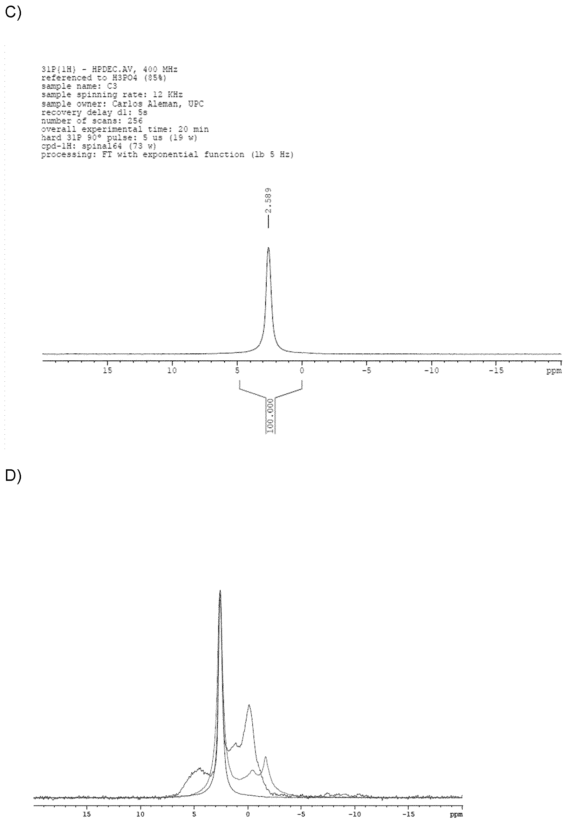

**Figure 18A-D.** Figure 18A shows the $^{31}$P spectrum of HAp sample as obtained by synthesis (Hap/p) where the co-existence of crystalline and disordered phase(s) (amorphous calcium phosphate) is present. The crystallinity is 43%. Weak signals are probably due to hydrogenphosphate and dihydrogenphosphate. Figure 18B shows the $^{31}$P spectrum of sintered HAp sample (Hap/s) where a re-organization is observed. The co-existence of several crystalline and disordered phase(s) (amorphous calcium phosphate) is also present. The crystallinity is 65%. Weak signals are probably due to hydrogenphosphate and dihydrogenphosphate. Figure 18C shows the $^{31}$P spectrum of HAp sample after permanent polarization according to the present invention (Hap/tsp) where the crystallinity is 76%. Weak signals are no longer present. Figure 18D shows the overlapping of spectra from figures 18A-C.

**Figure 19.** $^{1}$H (a), $^{13}$C (b) and $^{31}$P NMR (c) spectra of a set 1 sample (Table 4) obtained after reaction for 24 h at 95 °C and using a 5 mM zirconyl trichloride solution for preparing the layered system.

**Figure 20.** Variation of Gly/Phos (○) (Gly+Ala)/Phos (♦) and Gly/Ala (▲) ratios versus time for reactions performed at 95 °C using set1 samples (Table 4) prepared from a 5 mM zirconyl thrichloride solution (a), versus temperature for reactions performed during 24 h using the same sample (b) and versus concentration of zirconyl trichloride solutions (c) for reactions performed at 95 °C, 24 h and using set 1 samples.

**Figure 21.** High resolution XPS spectra for (a) p-cHAP, (b) p-cHAP + Phos-Zr-Phos, c) p-cHAP + Phos-Zr-Phos after negative reaction (e.g. without exposure to UV radiation) and d) p-CHAP + Phos-Zr-Phos after positive reaction (24 h at 195 °C): N1s, and Zr3d regions.

**Figure 22.** SEM micrograps of a set 1 sample before (a) and after (b) reaction for 24 h at 95 °C and using a 5 mM zirconyl trichloride solution for preparing the layered system.

**Figure 23.** a) FTIR spectra of a set 1 sample after reaction for 24 h at 95 °C and using a 5 mM zirconyl trichloride solution for preparing the layered system. Insets compare the 1700-1500 cm$^{-1}$ region for the above sample after (b) and before reaction (d), a set 2 sample after reaction (c) and a mixture of glycine and alanine (2:1 weight ratio) (e).

**Figure 24.** X-ray diffraction patterns corresponding to polarized c-HAp (a) and the set 1 sample before (b) and after (c) reaction for 24 h at 95 °C and using a 5 mM zirconyl trichloride solution for preparing the layered system. Gray crosses point out characteristic X-ray diffraction reflections of the catalyst that disappear after reaction whereas red crosses point out new reflections that can be observed after reaction. Circled symbols indicate the reflections that changed more drastically during reaction.

## Detailed description of the invention

The present disclosure relates to permanently polarized hydroxyapatite characterized in that its crystallinity is over 65%, preferably, over 70%, more preferably, over 75%, and its corresponding RMN $^{31}$P spectrum is as shown on figure 18C.

In the present invention, the term "permanently polarized" means that the hydroxyapatite has undergone a complete structural redistribution, almost perfect, with a high crystallinity degree, i.e. particularly with a low amount of amorphous calcium phosphate and the presence of vacancies detected by increased electrochemical activity and the accumulation of charge per unit mass and surface. It has an electrochemical activity and ionic mobility which do not disappear over. The chemical differences between the permanently polarized hydroxyapatite and the corresponding synthesized and sintered hydroxyapatite are shown on RMN $^{31}$P spectra according to figures 18A-C.

The present disclosure further relates to a composition or material comprising the permanently polarized hydroxyapatite as defined herein.

In a further embodiment, said composition or material further comprises at least one of the followings: silicates; biocompatible polymers, including but not limited thereto, polylactic acid (PLA), poly lactic-co-glycolic acid (PGLA), polyglycolide (PGA), polydioxanone (PDO), polyhydroxybutyrate (PHB) and collagen; and metal ions, preferably selected from Mg, Sr, Fe, Mn, Zr, Au, and Ti, more preferably Zr.

The present invention relates to a process for obtaining a permanently polarized hydroxyapatite comprising the steps of:

(a) obtaining sintered samples of crystalline hydroxyapatite and amorphous calcium phosphate;
(b) heating the samples obtained in (a) at a temperature between at least 1000°C and 1200°C;
(c) applying a constant DC voltage between 250 and 2500 V for at least 1 minute or an electrostatic discharge between 2500 and 1500000 V for less than 10 minutes;
(d) cooling the samples maintaining the DC voltage, preferably to room temperature.

The sintering step (a) is a thermal treatment of a ceramic at a temperature lower than its melting point. In the instant case, the sintering step is carried out at a temperature between 800-1100°C, preferably about 1000°C.

Also, importantly, the process of the present invention applies a constant DC voltage (see step (c)) and not a constant electric field as disclosed in the state of the art. When a constant DC voltage is applied the corresponding electric field is zero.

In a further embodiment, the constant voltage is applied in step (c) for 0.5 to 1.5 hours. In another embodiment, the constant voltage is applied in step (c) for about 1 hour.

The present disclosure relates to the use of the permanently polarized hydroxyapatite as defined herein or the composition or material comprising said permanently polarized hydroxyapatite as defined herein in biomedical applications. Preferably, said biomedical application is selected from cementum for teeth, bone, prosthesis, medical devices, drugdelivery, gene therapy and tissue regeneration.

The present disclosure further relates to the use of the permanently polarized hydroxyapatite as defined herein or the composition or material comprising said permanently polarized hydroxyapatite as defined herein as electrodes.

The present disclosure further relates to the use of the permanently polarized hydroxyapatite as defined herein or the composition or material comprising said permanently polarized hydroxyapatite as defined herein for doping polymers.

The present disclosure further relates to the use of the permanently polarized hydroxyapatite as defined herein or the composition or material comprising said permanently polarized hydroxyapatite as defined herein as catalysts, preferably as a photoelectrocatalyst or an electrocatalyst.

The present inventors have found that the permanently polarized hydroxyapatite as defined herein or the composition or material comprising said permanently polarized hydroxyapatite as defined herein, can be used as a component in a trilayered catalyst system based on (zirconium) amino tris(methylene phosphonic acid) which allows to catalyze the synthesis of natural amino acids, such as glycine and alanine. This synthesis takes place in the solid state with a significant yield and without rendering noticeable by-products as demonstrated by NMR spectroscopy. The reaction can be performed at a relatively low temperature (75-105 °C) and short time (e.g. less than 24 h) but exposition to UV radiation is indispensable. The catalyst is able to fix molecular nitrogen which acts as the nitrogen source and adsorb $CO_2$. Carbon dioxide and methane are involved in the production of carboxylic groups and both methylene and methyl groups, respectively. Water also affects the catalyst modifying its dielectric behaviour and contributing to ionic mobility. These results are very interesting since it is provided a new and clean synthesis process of amino acids that could proceed in the solid state, avoiding the dissolution of reactants in great water volumes as proposed in former prebiotic synthesis. The capacity of fixing molecular nitrogen and using a mildly reducing atmosphere ($N_2$, $CO_2$, $H_2O$ and $CH_4$) are also noticeable points of the new catalyst system. This surprising use opens the possibility of employing this catalyst family to get amino acids from a mildly reducing atmosphere (i.e. containing $H_2O$, $CH_4$, $N_2$ and $CO_2$,) instead of the less probable reducing atmosphere ($H_2O$, $CH_4$, $NH_3$ and $H_2$). Furthermore, the use of this catalyst by adsorbing $CO_2$ allows to obtain organic compounds (such as the production of amino acids as shown in the example section), while reducing the amount of $CO_2$ in the atmosphere which represents a clear contribution to the existing environmental problems due to high $CO_2$ volume concentrations in the atmosphere (green house effect).

This catalyst is based on the efficient zirconium amino tris(methylene phosphonic acid) trilayered system, abbreviated hereinafter as Phos-Zr-Phos. Nevertheless, the compound supporting the trilayered system should play a determinant rule to anchor properly the first phosphonate layer. This feature will be also evaluated comparing the results from a layered silicate (e.g. sodium montmorillonite), a layered aluminosilicate (e.g. mica) and a calcium phosphate compound (HAp, $(Ca_{10}(PO_4)_6(OH)_2)$) able to establish strong ionic interactions between its calcium ions and the deposited phosphonate layer. The application of a thermally stimulated polarization to HAp enhanced the electrochemical activity and stability and the electrical conductivity, while increased significantly the adsorption of phosphates and phosphonates (particularly the amino tris(methylene phosphonic acid, ATMP) with respect to non-treated (as synthesized) HAp particles. (See example section for further details about this process)

The present disclosure further relates to the use of the permanently polarized hydroxyapatite as defined herein or the composition or material comprising said permanently polarized hydroxyapatite as defined herein for adsorbing organic molecules. Preferably, said molecules are selected from carbohydrates, amino acids, lipids, DNA, RNA, biopolimers and ATP. More preferably, said biopolymers are selected from polylactic acid (PLA), poly lactic-co-glycolic acid (PGLA), polyhydroxybutyrate (PHB) and polydioxanone (PDO).

The present disclosure further relates to the use of the permanently polarized hydroxyapatite as defined herein or the composition or material comprising said permanently polarized hydroxyapatite as defined herein as a component in a solid-state battery. As used herein, a solid-state battery is a battery that has both solid electrodes and solid electrolytes. As a group, these materials are very good conductors of ions, which is necessary for good electrolyte and electrode performance, and are essentially insulating toward electrons, which is desirable in electrolytes but undesirable in electrodes. The high ionic conductivity minimizes the internal resistance of the battery, thus permitting high power densities, while the high electronic resistance minimizes its self-discharge rate, thus enhancing its charge retention.

The present disclosure further relates to the use of the permanently polarized hydroxyapatite as defined herein or the composition or material comprising said permanently polarized hydroxyapatite as defined herein as a component in an energy harvesting chip which is a chip that can generate their own energy. Energy harvesting is defined as the conversion of ambient energy into usable electrical energy. When compared with the energy stored in common storage elements, like batteries and the like, the environment represents a relatively inexhaustible source of energy. Consequently, energy harvesting (i.e. scavenging) methods must be characterized by their power density, rather than energy density.

The present invention is now further illustrated by reference to the following examples which do not intend to limit the scope of the invention.

## EXAMPLES

### *Process for obtaining permanently polarized HAp and ACP*

*Materials.* Ammonium phosphate dibasic [(NH$_4$)$_2$HPO4; purity $\geq$ 99.0%], ammonium hydroxide solution 30% (NH$_4$OH; purity: 28-30%), tetrasodium pyrophosphate $(P_2O_7^{4-})$, sodium triphosphate (polyP) and ATMP were purchased from Sigma-Aldrich. Calcium nitrate [Ca(NO$_3$)$_2$; purity $\geq$ 99.0%] was purchased from Panreac (Barcelona, Spain). Ethanol (C$_2$H$_5$OH; purity $\geq$ 99.5%) was obtained from Scharlab (Barcelona, Spain). Fetal bovine serum (FBS), for contact angle measurements, was purchased from Gibco.

*Synthesis of HAp and ACP.* A simple procedure was used to prepare ACP and cHAp samples, the only difference being the thermal post-treatment applied to the reaction mixture.[35] Reagent conditions were adjusted to get a Ca/P ratio of 1.67. For both ACP and cHAp, 15 mL of 0.5 M (NH$_4$)$_2$HPO$_4$ in de-ionized water (pH adjusted to 11 with an ammonia 30% w/w solution) were added drop-wise (rate of 2 mL·min$^{-1}$) and under agitation (400 rpm) to 25 mL of 0.5 M Ca(NO$_3$)$_2$ in ethanol. After that, the reaction mixture was stirred 1 h by agitation (400 rpm) at room temperature. In the case of ACP the resulting suspension was aged for 24 h at 37 °C, whereas hydrothermal conditions were applied during 24 h for cHAp. In the hydrothermal synthesis the crystal growth is performed in an apparatus consisting of a steel pressure vessel called an "autoclave", in which a nutrient is supplied along with water. In the instant case, the temperature was 150°C and the pressure was 200 bar.

In both cases, the precipitate was separated by centrifugation and washed sequentially with de-ionized water and a 60/40 v/v mixture of ethanol-water (twice). A white powder was recovered after freeze-drying. ACP and cHAp obtained using this procedure have been denoted *"as prepared"* samples, hereafter abbreviated ACP/p and cHAp/p, respectively. *Sintering and thermally stimulated polarization process.* Sintered cHAp and ACP samples, hereafter denoted cHAp/s and ACP/s, respectively, were prepared by heating the previously synthesized powders at 1000 °C for 2 h in air. After this, powders were uniaxially pressed at 620 MPa for 10 min to obtain dense discs suitable for characterization. The dimensions of these specimens were 10 mm of diameter $\times$ 1.68 mm of thickness.

In order to get thermally stimulated polarized ACP and cHAp (ACP/tsp and cHAp/tsp, respectively), discs of sintered samples were sandwiched between stainless steel (AISI 304) plates, heated to 1000 °C in air and, simultaneously, polarized for 1 h under application of a constant DC voltage (V). Subsequently, samples were cooled to room temperature, maintaining the DC voltage. Preliminary assays were performed using V values that ranged from 250 to 2000 V, the best results being obtained for 500 V. Accordingly, all experiments described in this work correspond to ACP/tsp and cHAP/tsp samples polarized using **V=** 500 V.

***Characterization** of the **permanently polarized HAp and ACP**

*X-Ray diffraction.* The crystallinity and structure was studied by wide angle X-ray diffraction (WAXD). Patterns were acquired using a Bruker D8 Advance model with Cu $K_\alpha$ radiation ($\lambda$ = 0.1542 nm) and geometry of Bragg-Brentano, theta-2 theta. A one-dimensional Lynx Eye detector was employed. Samples were run at 40 kV and 40 mA, with a 2-theta range of 10-60, measurement steps of 0.02°, and time/step of 2-8 s. Diffraction profiles were processed using PeakFit v4 software (Jandel Scientific Software) and the graphical representation performed with OriginPro v8 software (OriginLab Corporation, USA).

The crystallite size (L) in the direction perpendicular to the (211) planes was derived from X-ray diffraction profiles considering the (211) peak width and line broadening measurement using the Scherrer equation:[36]

$$L = \frac{0.9\lambda}{\beta \cos\theta} \qquad (1)$$

where $\lambda$ is the wavelength (CuK$_\alpha$), $\beta$ is the full width at half maximum height of the (211) peak, $\theta$ is the diffraction angle and 0.9 is a shape factor.

The crystallinity ($\chi_c$) was obtained using the following expression:[37]

$$\chi_c = 1 - \frac{V_{112/300}}{I_{300}} \qquad (2)$$

where $I_{300}$ is the intensity of the (300) reflection and $V_{112/300}$ is the intensity of the hollow between the (112) and (300) reflections, which disappears in non-crystalline samples.

*X-ray photoelectron spectroscopy (XPS).* XPS analyses were performed in a SPECS system equipped with a high-intensity twin-anode X-ray source XR50 of Mg/AI (1253 eV/1487 eV) operating at 150 W, placed perpendicular to the analyzer axis, and using a Phoibos 150 MCD-9 XP detector. The X-ray spot size was 650 $\mu$m. The pass energy was set to 25 and 0.1 eV for the survey and the narrow scans, respectively. Charge compensation was achieved with a combination of electron and argon ion flood guns. The energy and emission current of the electrons were 4 eV and 0.35 mA, respectively. For the argon gun, the energy and the emission current were 0 eV and 0.1 mA, respectively. The spectra were recorded with pass energy of 25 eV in 0.1 eV steps at a pressure below $6\times10^{-9}$ mbar. These standard conditions of charge compensation resulted in a negative but perfectly uniform static charge. The C1s peak was used as an internal reference with a binding energy of 284.8 eV. High-resolution XPS spectra were acquired by Gaussian-Lorentzian curve fitting after s-shape background subtraction. The surface composition was determined using the manufacturer's sensitivity factors.

*FTIR spectroscopy.* Infrared absorption spectra were recorded with a Fourier Transform FTIR 4100 Jasco spectrometer in the 1800-700 cm-1 range. A Specac model MKII Golden Gate attenuated total reflection (ATR) equipment with a heating Diamond ATR Top-Plate was used.

*Morphology.* Scanning electron microscopy (SEM) studies were carried out using a Focused Ion Beam Zeiss Neon40 microscope operating at 5 kV, equipped with an energy dispersive X-ray (EDX) spectroscopy system. Samples were deposited on a silicon disc mounted with silver paint on pin stubs of aluminum, and sputter-coated with a thin layer of carbon to prevent sample charging problems.

*Contact profilometry.* The surface roughness (Rq) of the prepared HAp discs was determined using a stylus profilometer (Veeco, Plainview, NY, USA).

*Contact angle.* Measurements were carried out using the sessile drop method at room temperature on an OCA 15EC with SCA20 software (Data-Physics Instruments GmbH, Filderstadt, Germany). The solvents used for these experiments were deionized water and FBS, contact angles being determined for both the first and second drop ($\theta$ and $\theta$, respectively). For $\theta$ measurements, the sessile drop was gently put on the surface of sample discs using a micrometric syringe with a proper metallic needle (Hamilton 500 $\mu$L). The ellipse method was used to fit a mathematical function to the measured drop contour. This procedure consists on approximate the drop contour to the line of an ellipse, deviations from the true drop shape being in the range of a few percent. The ellipse method provides accurate measure of the contact angle and holds the advantage that it is extremely fast. For each solvent, no less than ten drops were examined. Measures of $\theta$ were performed using the same procedure, even though an equilibration time of 1 min. was applied after depositing the second drop onto the first one.

*Determination of water content.* HAp discs were dried in an oven (100 °C) for 15 h. After this, samples reached the room temperature in a desiccator, being immediately weighted. Next, samples were immersed in deionized water for 1 hour. Samples were removed, patted dry with a lint free cloth, and weighted. The water content, expressed as increment in weight percent, was calculated as follows:

$$M_W(\%) = \frac{(W_W - W_D)}{W_D} \times 100 \qquad (1)$$

where Mw is the water content of the sample, Ww is the weight of the wet sample, and $W_D$ the weight of the dried sample. Ww and $W_D$ were determined using a Sartorius CPA26P analytical micro-balance.

*Cyclic voltammetry (CV).* The electrochemical behaviour was determined by CV using an Autolab PGSTAT302N equipped with the ECD module (Ecochimie, The Netherlands) with a three-electrode cell under a nitrogen atmosphere (99.995% in purity) at room temperature. A 0.1 M phosphate buffer saline solution (PBS; pH = 7.2 adjusted with NaOH) was used as the electrolyte in the three-electrode cell. The working compartment was filled with 30 mL of the electrolyte solution. Steel AISI 316 sheets of $1 \times 1.5$ cm$^2$ (thickness 0.1 cm) were used as both the working and the counter electrodes, and an Ag|AgCl electrode was used as the reference electrode which contained a KCl saturated aqueous solution (offset potential versus the standard hydrogen electrode, $E^0 = 0.222$ V at 25 °C). All potentials given in this report are referenced to this electrode. HAp discs prepared as described above were fixed on the working electrode using a two-side adhesive carbon layer. The initial and final potentials were -0.40 V, whereas a reversal potential of 0.80 V was considered. The scan rate was 50 mV/s.

The electroactivity, which indicates the ability to exchange charge reversibly, was evaluated by examining the similarity between the anodic and cathodic areas of the control voltammogram. The electrochemical stability (i.e. loss of electroactivity, LEA), which decreases with the oxidation and reduction areas of consecutive control voltammograms, was determined using the following expression:

$$LEA = \frac{\Delta Q}{Q_{II}} 100 \qquad (2)$$

where $\Delta Q$ is the difference of voltammetric charge between the second cycle and the last cycle and $Q_{II}$ is the voltammetric charge corresponding to the second cycle. In this work all values of *LEA* were referred to 1000 consecutive oxidation-reduction cycles.

The specific capacitance (SC; in F/g) of HAp in the electrode was calculated as:

$$SC = \frac{Q}{\Delta Vm} \qquad (3)$$

where Q is voltammetric charge, which is determined by integrating either the oxidative or the reductive parts of the cyclic voltammogram curve, $\Delta V$ is the potential window and m is the mass of polymer on the surface of the working electrode. The latter is derived from the productivity current and polymerization charge.[38]

*Electrochemical Impedance Spectroscopy (EIS).* EIS measurements were performed using an AUTOLAB PGSTAT302N in the 10 kHz to the 10 mHz frequency range and the amplitude of the sinusoidal voltage was 10 mV. All experiments were carried at room temperature. Appropriated sized films were pressed in a disc format and were sandwiched between two stainless steel electrodes (diameter = 1.5 cm) assembled into an isolating resin holder.[39] The cell was tightened with screws to ensure constant pressure fastening. Films thickness was between 1.68 and 2.00 mm determined by a micrometer and the area was about 1.766 cm$^2$. Prior analyses, samples were previously dried by heating at 100° C in an oven overnight. After data collection, EIS results were then processed and fitted to an electrical equivalent circuit (EEC).

*Adsorption onto treated cHAP.* The concentration of the adsorbate in the working solutions was 100 mM for $P_2O_7^{4-}$ and 200 mM for both polyP and ATMP, while the pH considered in this study was 7 in all cases. The concentration of $P_2O_7^{4-}$ was a half of that used for the other two adsorbates because of limitations in the solubility of the former specie. For the incubation, 500 μL of the working solution with the adsorbate were deposited onto 50 mg of cHAp. After overnight agitation at 25 °C, adducts were separated by centrifugation at 6500 rpm during 5 minutes at 4 °C. Sediments were re-suspended in distilled water. After this process, which was repeated two times, the obtained pellets were frozen at -80 °C for 3 h and, subsequently, the humidity was removed using a lyophilizer.

### *Chemical characterization and choice of samples for electrochemical and adsorption assays*

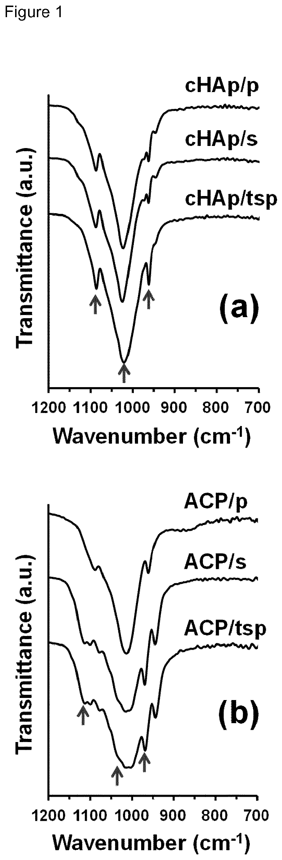

The FTIR spectra of the studied cHAp and ACP samples, which show typical $PO_4^{3-}$ bands at the region comprised between 950 and 1200 cm$^{-1}$, are compared in Figure 1. The spectra of cHAp/p, cHAp/s and cHAp/tsp show characteristic vibrational modes of $PO_4^{3-}$ at $\nu_1$= 962 cm$^{-1}$ and $\nu_3$= 1016, 1087 cm$^{-1}$, the resemblance between the three spectra indicating that cHAp/p does not undergo significant structural changes when sintered and polarized. In contrast, the apparition of new bands and shoulders (i.e. at 970 and 1037 cm$^{-1}$), as well as the shifts in the existing bands (i.e. from 963 and 1090 cm$^{-1}$ to 947 and 1098 cm$^{-1}$, respectively), in the spectra of ACP/s and ACP/tsp evidence important structural reorganizations in ACP/p after thermal and polarization treatment. Powder ACP samples heated at temperatures ranging between 600 and 1000° C were characterized by Raynaud *et al.*.[40] The apparition of new FTIR bands were attributed to the formation of a structure formed by cHAp and tricalcium phosphate (TCP) phases.

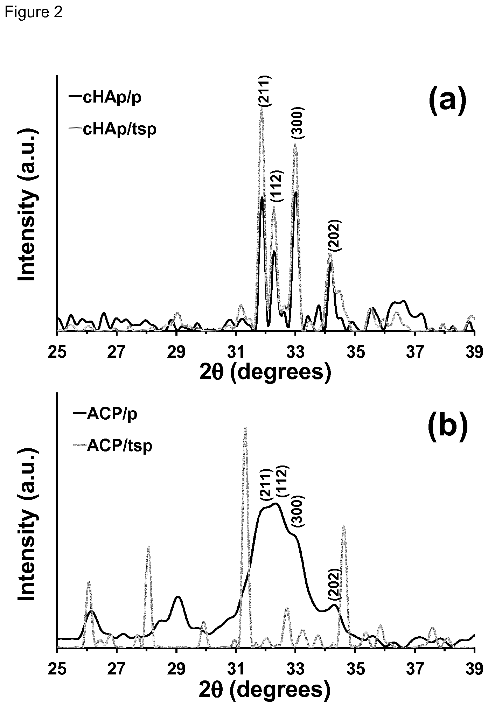

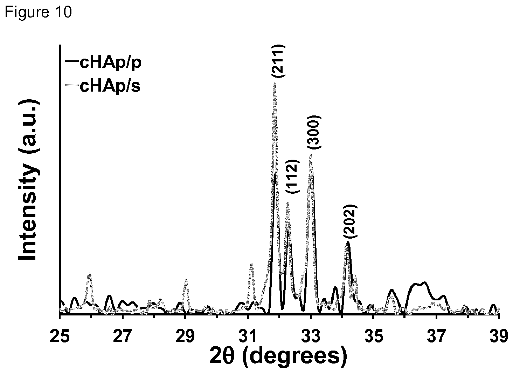

Structural analyses of cHAp and ACP particles by WAXD were focused on peaks at 2θ=32°-34°, which are characteristic of the (211), (112), and (300) HAp reflections. Although the comparison between the diffraction patterns recorded for cHAp/p and cHAp/tsp reveal small structural changes (Figure 2a), the thermally stimulated polarization process provokes important increments in both the crystallinity ($\chi_c$) and the crystallite size (*L*). Thus, the $\chi_c$ of cHAp/p and cHAp/tsp samples was 0.42±0.01 and 0.75±0.02, respectively, while the crystallite size of cHAp/tsp, L= 86±2 nm, was around 40% larger than that of cHAp/p (L= 61±2 nm). The variation of $\chi_c$ and L has been attributed to the formation of OH$^-$ defects. Fujimori *et al.*[41] reported that OH$^-$ ions scape from the HAp matrix above 800 °C, this dehydration process giving place to the formation of vacancies and O$^{2-}$ ions. In addition to the induction of a small amount of OH$^-$ defects, a monoclinic-to-hexagonal thermal phase transition occurs upon the application of such treatment.[42-44] The hexagonal phase becomes most stable at room temperature because of the order-disorder phase transition, which is accounted for by the change in the position of the OH$^-$ ions.[42-44] Although the structural differences between monoclinic and hexagonal HAP are small (Figure 2a) they are sufficient to exert a strong impact on some of its properties (see next subsections). The diffraction pattern recorded in this work for cHAp/s ($\chi_c$= 0.65±0.02 and L= 86±3 nm) is compared in Figure 10 with that of cHAp/p.

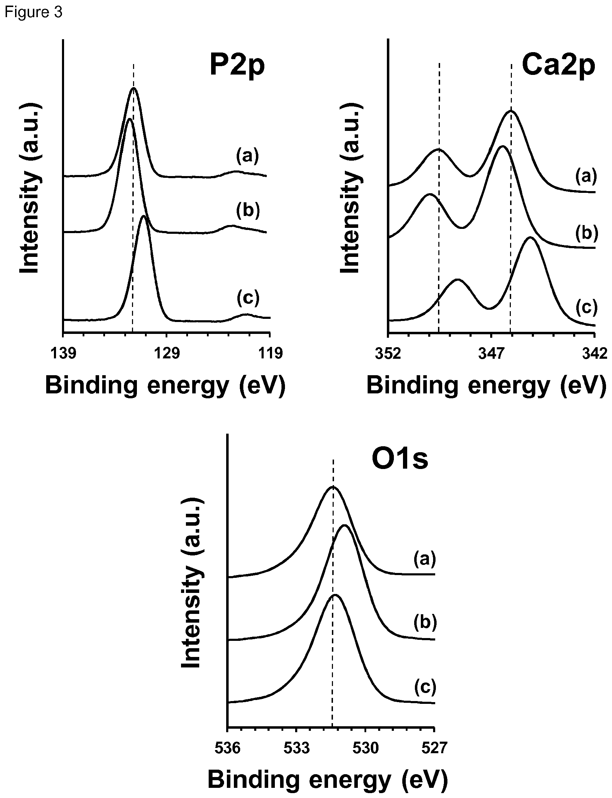

Figure 3 compares the characteristic XPS spectra in the P 2p, Ca 2p and O 1s regions for cHAp/p, cHAp/s and cHAp/tsp. For cHAp/p the single P2p peak centered at 132.2 eV, which originates from the $PO_4^{3-}$ anions,[45,46] undergoes a slight shift towards higher and lower energies ($\Delta_{BE}$= +0.4 and -1.0 eV) upon the application of sintering and thermally stimulated polarization treatment, respectively. The binding energies of the Ca 2p$_{3/2}$ and Ca 2p$_{1/2}$ peaks, which are detected at 346.1 and 349.6 eV, respectively, for cHAp/p,[45,47] experience shifts to 346.5 and 350.0 eV for cHAp/s and to 345.1 and 348.6 eV for cHAp/tsp. These variations are fully consistent with the existence of structural changes associated to phase transitions. Moreover, inspection of the chemical composition as determined by XPS, which is displayed in Table 1, is consistent with the formation of thermally-induced OH$^-$ vacancies. Thus, the content of oxygen is around 2 wt.% lower for cHAp/s and cHAp/tsp than for cHAp/p. Interestingly, the Ca/P molar ratio of the cHAp/p samples is very close to the stoichiometric value of 1.67. However, cHAp/s and cHAp/tsp experience a small reduction with respect to such ideal value, supporting the apparition of vacancies. On the other hand, the nitrogen found in cHAp/p, cHAp/s and cHAp/tsp, which ranges from 0.28 to 0.40 wt.%, has been attributed to the adsorption of N$_2$ from the atmosphere.

**Table 1.** Ca, P, O, Na and N concentration (wt %) and Ca/P molar ratios determined by XPS of cHAP/p, cHAp/s and cHAp/tsp samples before and after incubation in presence of $P_2O_7^{4-}$, polyP and ATMP.

| | Ca (wt.%) | P (wt.%) | O (wt.%) | Na (wt.%) | N (wt.%) | Ca/P (molar) |
|---|---|---|---|---|---|---|
| cHAp/p | 38.76 | 18.09 | 42.86 | 0.00 | 0.29 | 1.66 |
| cHAp/s | 39.76 | 19.01 | 40.95 | 0.00 | 0.28 | 1.62 |
| cHAp/tsp | 40.12 | 18.95 | 40.53 | 0.00 | 0.40 | 1.64 |
| cHAp/s+ $P_2O_7^{4-}$ | 39.67 | 22.76 | 31.67 | 5.58 | 0.32 | 1.59 |
| cHAp/s+polyP | 38.76 | 18.95 | 35.62 | 6.38 | 0.29 | 1.32 |
| cHAp/s+ATMP | 39.23 | 19.27 | 38.32 | 0.00 | 3.18 | 1.48 |
| cHAp/tsp+ $P_2O_7^{4-}$ | 39.54 | 22.56 | 25.64 | 11.91 | 0.35 | 1.35 |
| cHAp/tsp+polyP | 40.03 | 27.34 | 22.58 | 9.84 | 0.21 | 1.13 |

(continued)

| | Ca (wt.%) | P (wt.%) | O (wt.%) | Na (wt.%) | N (wt.%) | Ca/P (molar) |
|---|---|---|---|---|---|---|
| cHAp/tsp+ATMP | 39.12 | 24.08 | 32.72 | 0.00 | 4.08 | 1.26 |

Comparison of the diffraction patterns recorded for ACP samples as prepared and after conducting the thermally stimulated polarization process (ACP/p and ACP/tsp, respectively) is provided in Figure 2b. In this case, changes are very drastic, as is also reflected by the growth of $\chi_c$ and L from $0.05\pm0.02$ and $5\pm1$ nm for ACP/p to $0.74\pm0.03$ and $52\pm3$ nm for ACP/tsp. The structure exhibited by the crystalline fraction of ACP/p is identical to that observed for cHAp/p. However, the sintering process provokes the apparition of β-tricalcium phosphate (β-TCP: $β-Ca_3(PO_4)_2$) as the predominant phase. Although the high peaks at $2θ = 31.3°$ and $34.6°$ match well with those of the β-TCP card (#09-0169) in the Joint Committee on Powder Diffraction Standards *(JCPDS)*, the coexistence cHAp as a minor phase of ACP/tsp is probed by the persistent peak positions at $2θ= 31.9°$, $32.3°$, $33.0°$ and $34.3°$. These results suggest that the thermally stimulated polarization process induces partial decomposition of ACP/p, leading to the formation of β-TCP. A similar behavior was reported by different authors for sintered ACP (ACP/s) at 1100 °C (i.e. without applying any electric field),[5,47,48] and corroborated by our observation in the diffraction obtained for the samples prepared in this work by heating ACP/p to 1000 °C for 2 h in air (not shown). SEM micrographs displayed in Figure 10 reflect the drastic structural changes undergone by ACP/p samples when treated thermally and electrically.

Because of the predominance of the β-TCP phase in ACP/tsp transition, the rest of the present work (i.e. surface and electrochemical properties, as well as adsorption ability) has been focused on the comparison between cHAp/p and cHAp/tsp. For the sake of completeness, such comparison has been extended to sintered cHAp samples (named cHAp/s).

*Surface characterization*

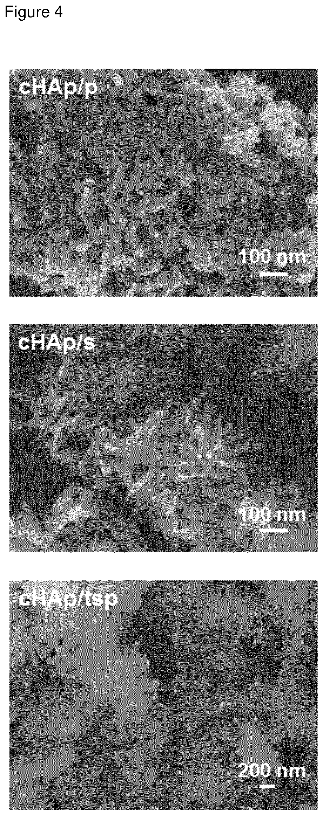

The surface morphologies of cHAp/p, cHAp/s and cHAp/tsp samples are compared in Figure 4. As it can be seen, SEM micrographs corroborate previously discussed WAXD results. cHAp/p samples are constituted by laminar crystals and fusiform rods, the same elements being also identified in cHAp/s and cHAp/tsp. However, the amount of such elements increases upon the application of external treatments, especially the thermal stimulation polarization. Thus, crystals are bigger in HAp/tsp than in cHAp/p and c/HAp/s, which is consistent with the $\chi_c$ variation discussed above. On the other hand, micrographs clearly reflect that the crystallite size increases with the increasing amount of crystals (i.e. WAXD results showed that L varies as follow: cHAp/tsp > cHAp/s > cHAp/p).

Table 2 indicates that, although the surface roughness (Rq) of cHAp/p samples remained practically unaltered upon the application of the polarization and/or thermal treatments, the surface energy changed considerably. The contact angle of water ($θ_{water}$) was ~4° for cHAp/p, cHAp/s and cHAp/tsp, indicating that the three are very hydrophilic materials, as it was expected because of their surface charge. In contrast, the contact angle in FBS ($θ_{FBS}$) was significantly lower for cHAp/s and cHAp/tsp than for cHAp/p (Table 2). This variation in the wetting suggests that the re-organization of the ions induced by the thermal and, especially, the polarization treatments increases the contribution of the polar component to the surface energy. In order to support the relative increase of the dispersive contribution with respect to the polar one, the contact angle of the second water and FBS drops ($θ_{wafer}$ and $θ'_{FBS}$, respectively) were determined for the three surfaces (see Methods section). Although the surfaces were less wetted than with the first drop, the behavior was practically identical to that described above (Table 2). Thus, the three hydrophilic materials led to very similar $θ_{water}$ values while the differences among $θ'_{FBS}$ values were similar to those obtained for $θ_{FBS}$.

**Table 2.** Roughness (Rq), contact angle of the first and second water drops ($θ_{water}$ and $θ'_{water}$), contact angle of the first and second FBS drops ($θ_{FBS}$ and $θ'_{FBS}$), and water content after immersion in deionized water (Mw) determined for cHAP/p, cHAp/s and cHAp/tsp samples.

| Sample | Rq (nm) | $θ_{water}$ (°) / $θ'_{water}$ (°) | $θ_{FBS}$ (°) / $θ'_{FBS}$ (°) | $M_W$ (%) |
|---|---|---|---|---|
| cHAp/p | $851\pm194$ | $3\pm1$ / $6\pm1$ | $81\pm2$ / $96\pm2$ | - |
| cHAp/s | $863\pm158$ | $4\pm1$ / $4\pm1$ | $61\pm2$ / $71\pm2$ | $7\pm1$ |
| cHAp/tsp | $882\pm92$ | $4\pm1$ / $4\pm1$ | $51\pm2$ / $62\pm2$ | $13\pm1$ |

In order to complement this information, water absorption assays were performed using the procedure described in the Methods section (Eqn 1). Unfortunately, cHAp/p discs broke immediately after water immersion, no measurement being possible in that case. However, the water content determined for cHAp/s and cHAp/tsp samples after immersion in deionized water, which is displayed in Table 2, were fully consistent with the $θ_{FBS}$ and $θ'_{FBS}$ values. Accordingly, water adsorption was 5% higher for HAp/tsp than for HAp/s.

*Electrochemical and electrical properties*

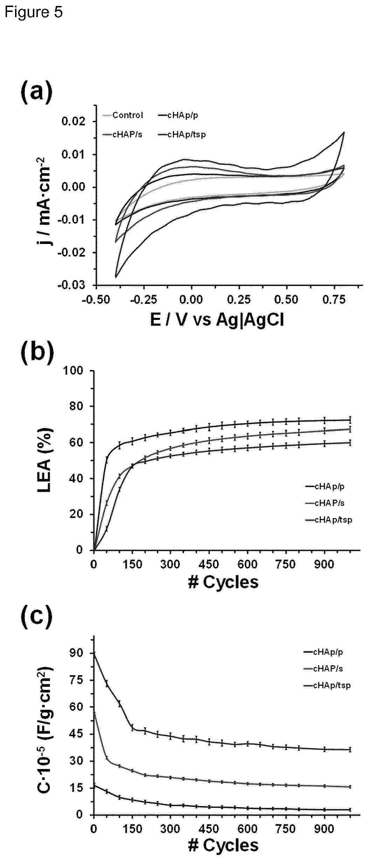

Cyclic voltammograms recorded in PBS for cHAp/p, cHAp/s and cHAp/tsp fixed on steel are compared in Figure 5a. As it can be seen, the electrochemical activity of cHAp/p is higher than that of steel, which was used as a control. However, the electroactivity increases considerably with thermal and electrical treatments (i.e. 46% and 150%, respectively). In the case of cHAp/tsp, such evident effect is accompanied of a significant enhancement of the anodic current intensity at the reversal potential. This behavior suggests that the structural changes provoked by the thermally stimulated polarization treatment facilitate the diffusion of ions through the inorganic matrix and, therefore, the electrochemical response upon oxidation-reduction processes.

Treatments also affect the electrostability, as is reflected by the variation of the LEA (Eqn 2) with the number of consecutive oxidation-reduction cycles (Figure 5b). As it can be seen, in all cases the electrochemical stability decreases rapidly during the first 100-150 redox cycles, the reduction of the LEA being considerably slower along the next cycles. After 1000 cycles, the electroactivity decreased 72%, 67% and 60% for cHAp/p, cHAp/s and cHAp/tsp, respectively, evidencing that structural changes caused by thermally stimulated polarization process also enhances the stability of the electrochemical properties. The behavior followed by the specific capacitance (C in Eqn 3) is fully consistent with that of the electroactivity. Thus, although C is very small in all cases, the ability to store charge of cHAp/p (C= $16 \cdot 10^{-5}$ $F/g \cdot cm^2$) is 71% and 82% smaller than those of cHAp/s and cHAp/tsp (C= $56 \cdot 10^{-5}$ and $89 \cdot 10^{-5}$ $F/g \cdot cm^2$, respectively). The variation of the specific capacitance with the number of redox cycles (Figure 5c) was similar to that described above for LEA.

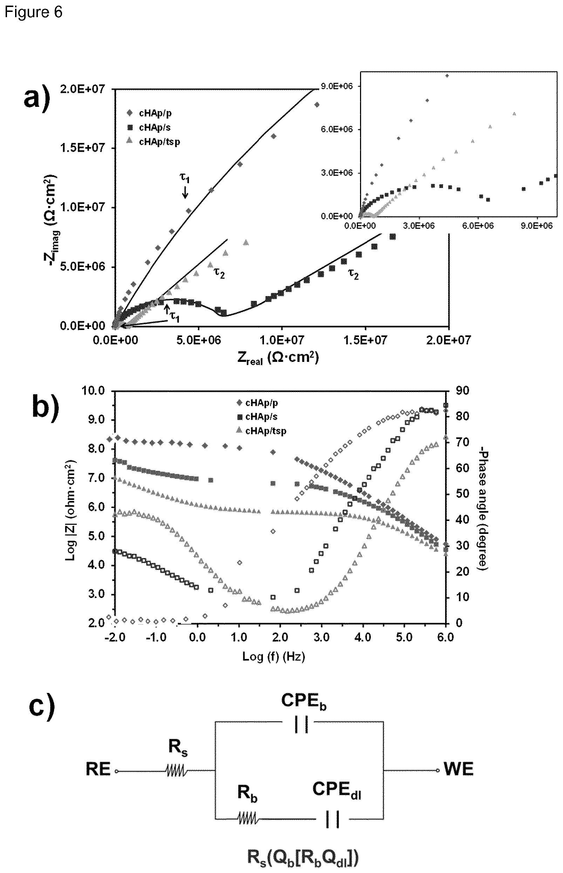

EIS measurements were carried out to evaluate the ionic conductivity inside the prepared HAp samples. Thus, this technique will provide information about the influence in the electrical properties of the inner interfaces created inside the material by the thermally stimulated polarization process. Figure 6 compares representative Nyquist plots obtained for cHAp/p, cHAp/s and cHAp/tsp. In a Nyquist plot, the first semi-circular response corresponds to the electron transfer resistance at the higher frequency range, which controls the electron transfer kinetics of the redox probe on the electrode-solid disk interface. The diameter of the semi-circle defines the resistance of electron transfer, usually called bulk resistance ($R_b$). The Nyquist plot recorded for cHAp/p (Figure 6a) exhibits only one dielectric relaxation time ($\tau$), which corresponds to a single charge transfer across the solid disk, indicating that the material has a high bulk resistance (i.e. low ionic conductivity) in the dry state. Bode plots (Figure 6b) show phase angles close to 80°, which correspond to resistive materials in the dry state. The semi-circle diameter in Nyquist plots (Figure 6a) is considerably smaller for cHAp/s and, especially, for cHAp/tsp, even though a second time constant appears. This feature has been attributed to a significant structural modification inside the HAp crystals that allows fast transport of charge across the disk. According to WAXD and SEM observations, cHAp/s and cHAp/tsp samples present higher more concentration of crystals as well as bigger crystals than cHAp/p. Therefore, the thermal treatment step promotes the growing of the crystal, while the thermally stimulated polarization treatment is that responsible is responsible of the definition of good pathways for charge transportation. This is reflected in the numerical evaluation of the EIS results (Table 3).

**Table 3.** Data of EIS results obtained from the electrical equivalent circuit (EEC) showed in the Figure 6c for cHAp/s and cHAp/tsp dry discs[a] after exposure to several treatment processes and after the phosphates inorganic molecules adsorption.

| Samples | $R_b$ ($\Omega$ cm$^2$) | $Q_{dl}$ (F cm$^{-2}$ s$^{n-1}$) | n | $Q_b$ (F cm$^{-2}$ s$^{n-1}$) | n |
|---|---|---|---|---|---|
| cHAp/p [a] | 134.6 M | - | - | $8.180 \times 10^{-10}$ | 0.76 |
| cHAp/s | 6.43 M | $1.248 \times 10^{-8}$ | 0.77 | $1.215 \times 10^{-5}$ | 0.44 |
| cHAp/tsp | 0.67 M | $4.558 \times 10^{-7}$ | 0.71 | $4.863 \times 10^{-5}$ | 0.55 |
| cHAp/s + polyP | 0.42 M | $5.076 \times 10^{-8}$ | 0.81 | $1.573 \times 10^{-5}$ | 0.43 |
| cHAp/s + $P_2O_7^{4-}$ | 1.00 M | $3.647 \times 10^{-8}$ | 0.73 | $1.309 \times 10^{-5}$ | 0.50 |
| cHAp/s + ATMP | 0.95 M | $2.159 \times 10^{-8}$ | 0.76 | $1.009 \times 10^{-5}$ | 0.42 |
| cHAp/tsp + polyP | 66.7 k | $5.550 \times 10^{-8}$ | 0.81 | $7.792 \times 10^{-4}$ | 0.63 |
| cHAp/tsp + $P_2O_7^{4-}$ | 0.35 M | $1.373 \times 10^{-8}$ | 0.79 | $3.812 \times 10^{-5}$ | 0.49 |
| cHAp/tsp + ATMP | 69.9 k | $5.699 \times 10^{-8}$ | 0.73 | $5.204 \times 10^{-5}$ | 0.48 |

[a] The EEC for cHAp/p is $R_s(R_bQ_b)$.

The electrical equivalent circuit (EEC) used to fit the experimental data is shown in Figure 6c. The EEC contains three important elements: $R_b$ that represents the bulk resistance; and $Q_b$ and $Q_{dl}$ that describes the ideal capacitances from both the cHAp thick film and double layer between the metal-disk surfaces, respectively. $R_S$ corresponds to the electrolyte solution resistance, even though it was considered ~0 $\Omega \cdot cm^2$ due to the absence of liquid electrolyte. The

$CPE_b$ real capacitance accounts for the non-uniform diffusion among the films adhered to the electrode surface. The $CPE_{dl}$ real capacitance is typically associated to the surface reactivity, surface heterogeneity and roughness, which in turn are related to the electrode geometry and porosity. Also, the CPE impedance, which has been expressed as $Z_{CPE}$ = $[Q (j\omega)^n]^{-1}$, represents an ideal capacitor and a pure resistor for n= 1 and n= 0, respectively, while it is associated with a diffusion process when n~ 0.5. All impedance data displayed in Figure 6a were fitted with the EEC showed in the Figure 6c, with exception of those obtained for cHAp/p. For EEC used the latter samples does not have the capacitance response from the double layer film and corresponds to $[R_s(R_bQ_b)]$.

According to Table 3, the $R_b$ is very low ($6.7 \times 10^5$ $\Omega \cdot cm^2$) for the cHAp/tsp sample compared to the cHAp/s one ($6.4 \times 10^6$ $\Omega \cdot cm^2$), which indicates that the ionic conductivity increased by one order of magnitude when the thermal treatment is combined with the polarization one. Another relevant change is the appearance of a second time constant ($\tau$) when larger crystals were obtained and these crystals were polarized at 500 V (Figure 6a). This feature indicates the creation of charge pathways inside the solid, which is reflected by the $CPE_b$. The last observation is in perfect agreement with both SEM micrographs and the electrochemical response determined by CV. According to Chaudhuri and co-workers,[49] the conductive sites in dry HAp should be considered as the channels along which ions are able to move by thermally activated hopping (such as the columnar OH⁻ ions or protons) while the capacitive sites are immobile ions. In contrast, Lukic *et al.*,[50] who found that the conductivity of HAp increases with temperature, attributed this behavior to geometric factors as growing of grain size. Liu and Shen[51] showed extensive dehydroxylation during the sintering of cHAp above 900 °C, OH- ions being responsible of the conductivity at high temperatures (i.e. in the range of 700-1000 °C).

### *Adsorption of pyrophosphate, triphosphate and trisphosphonate*

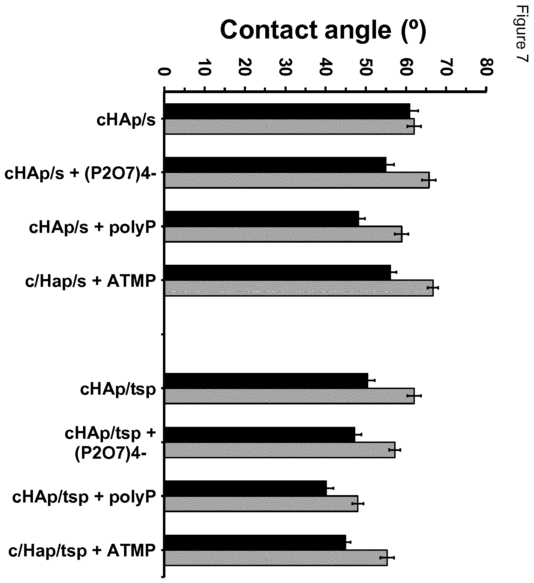

In a very recent study we examined the adsorption of $P_2O_7^{4-}$, polyP and amino-ATMP onto cHAp/p.[12] In order to examine how both thermal and electric treatments affect the adsorption of the same inorganic compounds, a complete study has been carried using cHAp/s and cHAp/tsp samples as substrates. According to our previous work, the concentration of adsorbate in the working solutions was 100 mM for $P_2O_7^{4-}$ and 200 mM for both polyP and ATMP, which provided clear adsorption signals for cHAp/p at pH 7. Figure 7a compares the contact angles for both the first and second FBS drop ($\theta_{FBS}$ and $\theta'_{FBS}$, respectively) determined for cHAp/s and cHAp/tsp before and after incubation in presence of the inorganic adsorbates. As it can be seen, the FBS-wettability of the two substrates increased upon incubation, suggesting that the three inorganic adsorbates were successfully adsorbed. Moreover, the reduction of the contact angle with the adsorbate followed the same variation for the two cHAp substrates: polyP < $P_2O_7^{4-}$ ≈ ATMP. Accordingly, the surface energy becomes higher upon the adsorption of polyP than upon the adsorption of $P_2O_7^{4-}$ and ATMP, independently of the treatment applied to the cHAp particles.

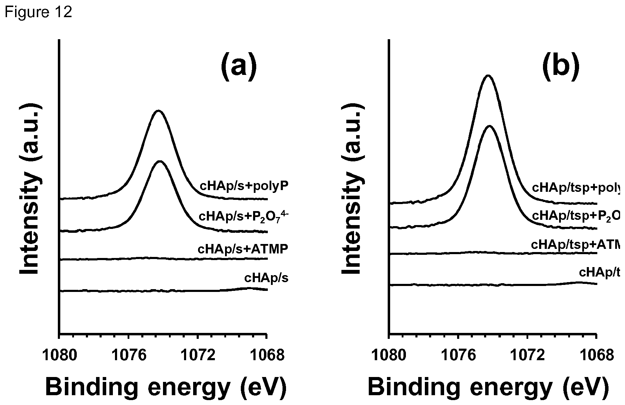

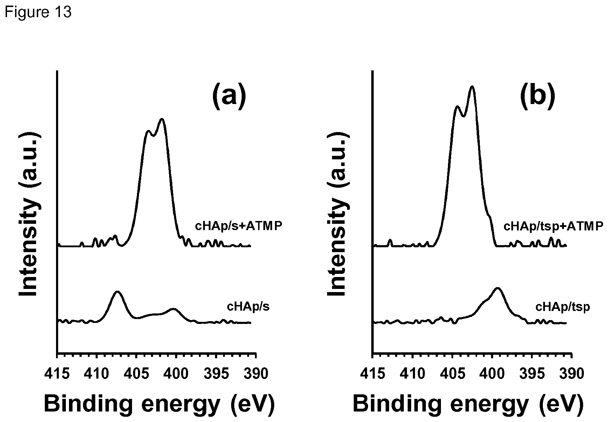

Adsorption of $P_2O_7^{4-}$, polyP and ATMP was also examined by using XPS. Comparison of the characteristic XPS spectrum in the Na1s region for cHAp/s and cHAp/tsp before and after incubation in presence of inorganic adsorbates reveals a peak centered at 1074.2 eV for samples treated with $P_2O_7^{4-}$ and polyP (Figure 12). This signal, which is identical to those reported by Gaskell et *al.*[52,53] for $Na_4P_2O_7 \cdot 10H_2O$ and $Na_5P_3O_{10}$, corroborates the incorporation of these compounds onto the surface of the two treated cHAp. In contrast the content of Na in non-incubated samples and samples incubated in presence of ATMP is null (Table 1). The ratios obtained using the Na1s atomic percent compositions indicate that the adsorption of $P_2O_7^{4-}$ and polyP is, respectively, ~2 and ~1.5 times higher for cHAp/tsp than for cHAp/s. A similar strategy was followed to identify the adsorption of ATMP, which is clearly detected through the peaks at the N1s region (Figure S4). Thus, the content of N in non-incubated samples and samples incubated in presence of $P_2O_7^{4-}$ and polyP is ≤ 0.40 wt.%, increasing to 3.18 and 4.08 wt.% for cHAp/s and cHAp/tsp samples incubated in presence of ATMP (Table 1). Assuming that the amount of $N_2$ adsorbed from the atmosphere is the same for incubated and non-incubated samples, the adsorption of ATMP is ~1.4 times higher for cHAp/tsp than for cHAp/s. The two peaks detected at 404.3 and 402.5 eV (Figure 13) for the latter samples have been attributed to nitrogen atoms of ATMP with different chemical environments (i.e. free and hydrogen bonded).[54]

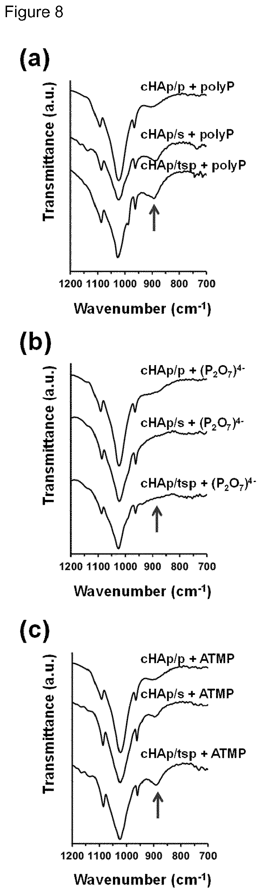

Figure 8 compares the FTIR spectra of cHAp/p, cHAp/s and cHAp/tsp after incubation in solution with $P_2O_7^{4-}$, polyP and ATMP at neutral pH. FTIR spectra of $P_2O_7^{4-}$, polyP and ATMP were reported in our previous work.[12] For

polyP, the weak shoulder identified at around 890 cm$^{-1}$ for cHAp/p (Figure 8a), which corresponds to the P-O-P asymmetric stretching, transforms into a well-defined adsorptions band for cHAp/s and, especially, cHAp/tsp. This feature is fully consistent with XPS observation, corroborating that the application of thermal and thermally stimulated polarization processes enhance significantly the ability of cHAp to adsorb polyP. Based on the FTIR spectra presented in Figures 1 and 8, the ability of cHAp samples to adsorb polyP was estimated using the ratio of integrated area of the peak at 1016 cm$^{-1}$ (belonging to the mineral) and the integrated area of the peak at 890 cm$^{-1}$ (belonging to polyP). Results indicated that the adsorption of polyP onto cHAp/p was 2.0 and 2.6 times lower than onto cHAp/s and cHAp/tsp, respectively, which is in good agreement with XPS results.

Unfortunately, this feature was much less clear for $P_2O_7^{4-}$. Thus, the band at 890 cm$^{-1}$ remained undetectable in the spectra displayed in Figure 8b, where the only evidence of adsorption is the very weak shoulder at 740-750 cm$^{-1}$ for cHAp/s and cHAp/tsp that has been attributed to the P-O-P symmetric stretching. It should be noted that the atomic percent content of Na1s detected by XPS in cHAp samples incubated with polyP is considerably higher than in those incubated with $P_2O_7^{4-}$ (Table 1), which is consistent with FTIR observations. Also, previous quantum mechanical calculations considering the (100) and (001) surfaces of cHAp evidenced that the adsorption of polyP is favored with respect to that of $P_2O_7^{4-}$.[18] Thus, the ability of the adsorbate to adapt its geometry to the crystallographic positions of the ions at the cHAp surfaces increases with the size of phosphate chain. Therefore, adsorbed $P_2O_7^{4-}$ was found to be significantly strained in comparison to adsorbed polyP.

FTIR results for the different cHAp samples incubated with ATMP (Figure 8c) reveals similar trends to those observed for polyP. Thus, the shoulder identified for cHAp/p at 900 cm$^{-1}$, which corresponds to asymmetric vibrations of alkylphosphonic,[55] transforms into a relatively intense and well defined peak for cHAp/s and, especially, cHAp/tsp. This variation is in agreement with XPS results, indicating that the ability of the different cHAp samples to adsorb ATMP increase in the following way: cHAp/p < cHAp/s < cHAp/tsp. The adsorption of ATMP onto of cHAp/s and cHAp/tsp was estimated to be, respectively, 2.2 and 3.0 times higher than onto cHAp/p, supporting XPS data.

*Adsorption-induced electrochemical protection and enhanced electrical conductivity*

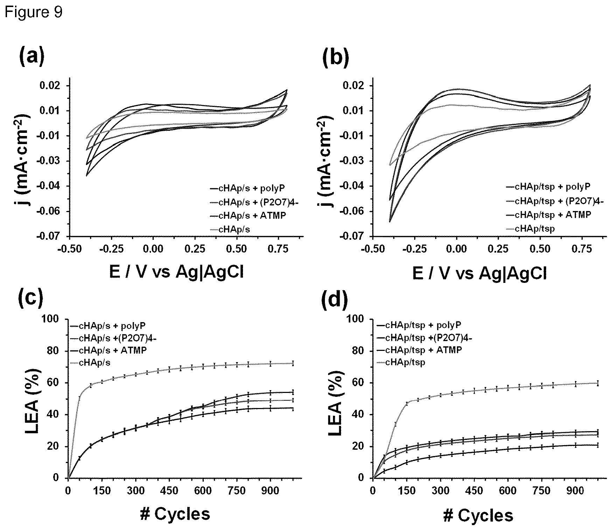

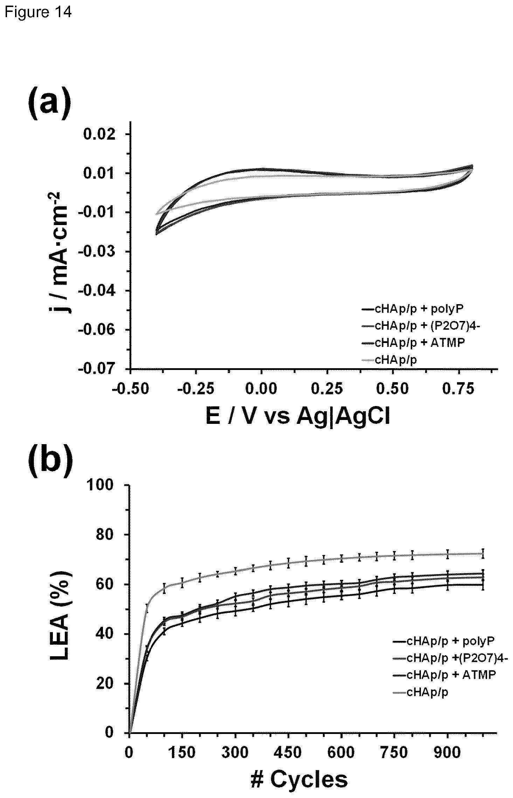

Cyclic voltammograms recorded for cHAp/p incubated in presence of polyP, $P_2O_7^{4-}$ and ATMP (Figure 14a) are very similar to those displayed in Figure 5a, suggesting that the amount of adsorbate at the mineral surface is not enough to alter the redox behavior. In contrast, cyclic voltammograms of incubated cHAp/s and, especially, cHAp/tsp are considerably different from those of non-incubated samples. This is clearly reflected in Figures 9a and 9b, which compares the voltammograms recorded for incubated and non-incubated samples. Thus, the electroactivity of incubated cHAp/s and cHAp/tsp samples is higher than that of non-incubated samples by ~60% and ~40%, respectively, suggesting that adsorbed molecules facilitates the exchange of ions between the mineral matrix and the PBS electrolyte solution during the oxidation and reduction processes.

However, the most striking feature refers to the variation of the electroactivity against the number of redox cycles. Thus, comparison of the LEA (Eqn 2) measured for incubated and non-incubated cHAp/p (Figure 14b) indicates that the electrochemical stability of the latter is lower (~10%) than that of samples with adsorbed polyP, $P_2O_7^{4-}$ or ATMP. This feature, which suggests that adsorbate molecules provide electrochemical protection to the mineral, is significantly enhanced for cHAp/s and cHAp/tsp, as is evidenced in Figures 9c and 9d, respectively. Thus, after 1000 redox cycles the loss of electroactivity of non-incubated cHAp/s and cHAp/tsp is higher than those of incubated samples by ~20% and ~25%, respectively. The LEA values of incubated cHAp/tsp are particularly striking (i.e. 21%, 27% and 29% for polyP, $P_2O_7^{4-}$ and ATMP, respectively). These low values evidence that the application of the thermally stimulated polarization treatment enhances not only the adsorption capacity but also improves the electrochemical activity and stability.

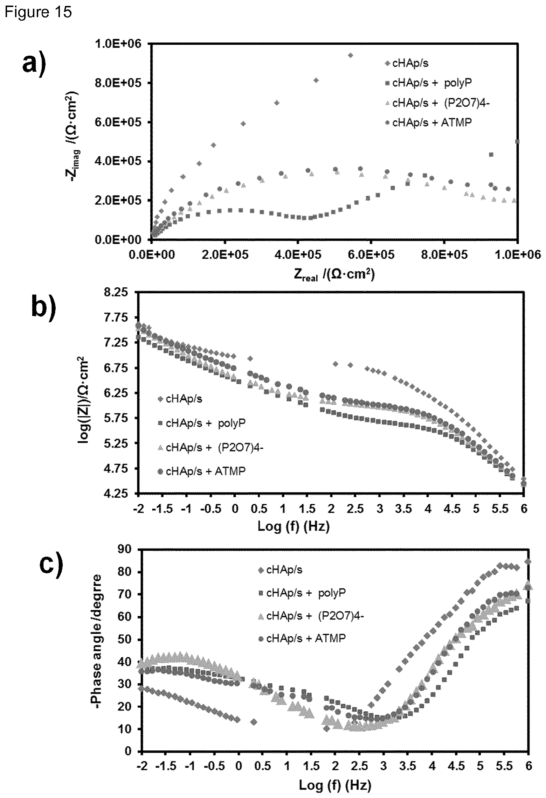

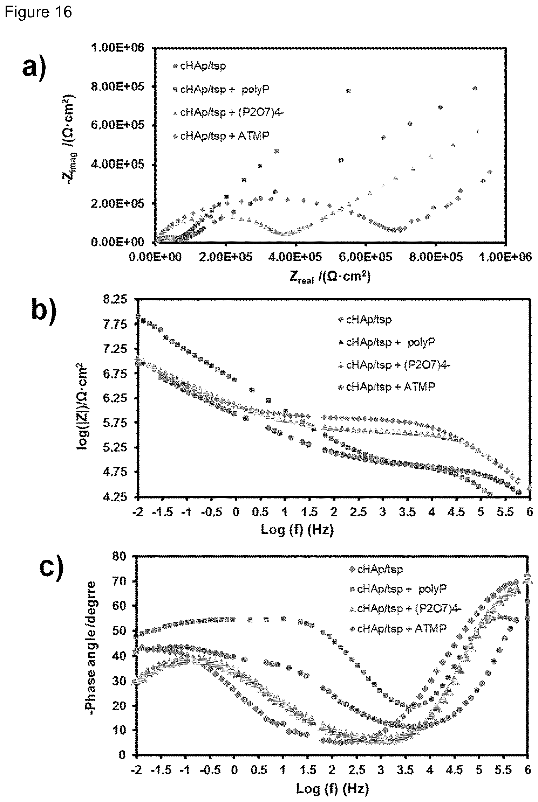

EIS results (Table 3) reflect the positive effects of adsorbed PolyP and ATMP in the ionic conductivity of treated cHAp samples in comparison to adsorbed $P_2O_7^{4-}$. This phenomenon is particularly remarkable for cHAp/tsp, which display the lowest bulk resistance (66.7 and 69.9 kΩ·cm$^2$ for samples with adsorbed PolyP and ATMP, respectively), evidencing that PolyP and ATMP promote the electron charge mobility inside the dry film. Thus, structural changes produced by the thermally stimulated polarization treatment favors the interaction of the mineral with both PolyP and ATMP, forming better charge transfer channels. The alignment of the OH$^-$ ions along the c-axis in cHAp/tsp samples seems to play a crucial role in the formation of such interaction. Figures 15 and 16 compare the Nyquist and Bode plots recorded for cHAp/s and cHAp/tsp, respectively, with the three examined adsorbates.

***Particular use of permanently polarized hydroxyapatite** as a **catalyst component in the synthesis of amino acids***

*Synthesis of amorphous (aHAp) and crystalline hydroxyapatite (cHAp)*

15 mL of 0.5 M $(NH_4)_2HPO_4$ in de-ionized water (pH adjusted to 11 with an ammonia 30 *w/w-%* solution) were added drop-wise (rate of 2 mL·min$^{-1}$) and under agitation (400 rpm) to 25 mL of 0.5 M $Ca(NO_3)_2$ in ethanol. After that, the reaction mixture was stirred 1 h by agitation (400 rpm) at room temperature. The suspension was aged for 24 h at 37 °C to get aHAP, whereas a hydrothermal treatment (200 bar at 150 °C for 24 h) was subsequently applied to get cHAp. The precipitate was separated by centrifugation and washed sequentially with de-ionized water and a 60/40 *v/v* mixture of ethanol-water (twice). A white powder with the theoretical Ca/P ratio of 1.67 was recovered after freeze-drying.

*Sintering process*

aHAp, cHPAp and montmorillonite powders were subsequently sintered by firstly heating them in a laboratory furnace (Carbolite ELF11/6B/301) at 1000 °C during 2 h at an air atmosphere and finally uniaxially pressed at 620 MPa for 10 min. Discs of 100 mm of diameter and 1.7 mm of thickness were finally obtained.

*Thermally stimulated polarization process*

In order to get thermally stimulated polarized HAp, Nanofil 757 and LM systems, the corresponding discs samples were sandwiched between stainless steel (AISI 304) plates, heated in the furnace to 1000 °C in air and, simultaneously, polarized for 1 h under application of a constant DC voltage of 500 V, which was previously reported as the optimal one for adsorption assays performed with polarized HAp.[2] Polarized samples will be named as p-cHAp, p-aHAp, p-N757 and p-LM. It should be pointed out that HAp could not be polarized if the sample was not previously sintered since the disk had not the sufficiently consistence and broke during the polarization process.

*Deposition of phosphonate and zirconate layers*

A trilayered system consisting in the succesive deposition of ATMP, zirconyl chloride and ATMP layers onto the appropriate substrate (i.e. mica, sintered aHAp and cHAp or silicate before and after being submitted to the polarization process) was obtained by immersion in the corresponding aqueous solutions at room temperature for 5 h. Concentrations of ATMP solutions to get the first and second AMTP layers were 5 mM and 1.25 mM, respectively, whereas the concentration of zirconyl chloride was varied in the different experiments (i.e. from 1 mM to 10 mM, respectively). After each immersion the samples were dried at 37 °C for 3 h. Fort the sake of completeness bilayered and monolayered systems (i.e. Pho-Z, Pho, Z) were also considered.

*Synthesis of amino acids*

A high pressure stainless steel reactor was employed to perform the synthesis of amino acids (AAs). The designed reactor was dotted with a manometer, an electric heater with a thermocouple and an external temperature controller. The reactor was also characterized by an inert reaction chamber of teflon (120 mL) where catalyst and water were incorporated, three independent inlet valves for $N_2$, $CH_4$, $CO_2$ and an outlet valve to recover the gaseous reaction products. An UV lamp (GPH265T5L/4, 253.7 nm) was also placed in the middle of the reactor to irradiate directly the solid sample, being the lamp protected by a UV transparent quartz tube. This was coated with a thin film of teflon in order to avoid any contact between the reaction medium and the silicate and therefore to discard other catalyst effects.

Reactions were performed in the 75-105 °C temperature range for reaction times between 2 and 96 h. Solid samples weighted approximately 150 mg and 0.5 ml of de-ionized liquid water were initially incorporated in the reaction chamber if it was considered necessary. The chamber was extensively purged with the first selected gas in order to eliminate the initial air content (i.e. $N_2$ or $CO_2$). Each selected gas was introduced to increase the reaction chamber pressure (measured at room temperature) in two or three atmospheres (i.e. the final pressure at room temperature was always 6 atm).

*Measurements*

Synthesis of amino acids was routinely verified by the ninhydrin (2,2-dihydroxyindane-1,3-dione) detection test for primary amines. To this end 0.5 mg of the solid recovered after reaction was immersed in a tube containing 0.2 *w/v*-% solution of ninhydrin in acetone and subsequently heated to 75 °C in an oven. The development of purple coloured solutions indicated the formation of the 2-(1,3-dioxoindan-2-yl)iminoindane-1,3-dione chromophore. Yellow-orange coloured solutions were on the contrary characteristic of the Schiff base generated by reaction with secondary amines, while uncoloured solutions derived from tertiary amines such as ATMP.

NMR spectra were acquired with a Bruker Avance III-400 spectrometer operating at frequencies of 400.1 MHz, 100.6, and 161.9 for $^1$H, $^{13}$C and $^{31}$P, respectively. Chemical shifts for $^1$H and $^{13}$C were calibrated using tetramethylsilane as an internal standard. Samples were dissolved in deuterated water containing 100 mM of HCl and 50 mM of NaCl.

X-ray photoelectron spectroscopy (XPS) analyses were performed in a SPECS system equipped with a high-intensity twin-anode X-ray source XR50 of Mg/Al (1253 eV/1487 eV) operating at 150 W, placed perpendicular to the analyzer axis, and using a Phoibos 150 MCD-9 XP detector. The X-ray spot size was 650 $\mu$m. The pass energy was set to 25 and 0.1 eV for the survey and the narrow scans, respectively. Charge compensation was achieved with a combination of electron and argon ion flood guns. The energy and emission current of the electrons were 4 eV and 0.35 mA, respectively. For the argon gun, the energy and the emission current were 0 eV and 0.1 mA, respectively. The spectra were recorded with pass energy of 25 eV in 0.1 eV steps at a pressure below $6 \times 10^{-9}$ mbar. These standard conditions of charge compensation resulted in a negative but perfectly uniform static charge. The C1s peak was used as an internal reference with a binding energy of 284.8 eV. High-resolution XPS spectra were acquired by Gaussian-Lorentzian curve fitting after s-shape background subtraction. The surface composition was determined using the manufacturer's sensitivity factors.

Scanning electron microscopy (SEM) studies were carried out using a Focused Ion Beam Zeiss Neon40 microscope operating at 5 kV, equipped with an energy dispersive X-ray (EDX) spectroscopy system. Samples were deposited on a silicon disc mounted with silver paint on pin stubs of aluminum, and sputter-coated with a thin layer of carbon to prevent sample charging problems.

Infrared absorption spectra were recorded with a Fourier Transform FTIR 4100 Jasco spectrometer in the 1800-700 cm-[1] range. A Specac model MKII Golden Gate attenuated total reflection (ATR) equipment with a heating Diamond ATR Top-Plate was used.

X-ray powder diffraction patterns were obtained in the beamline BL11-NCD at ALBA synchrotron (Cerdanyola del Vallés, Barcelona, Spain), by using a wavelength of 0.100 nm and an WAXS LX255-HS detector from Rayonix which was calibrated with diffractions of standard of a $Cr_2O_3$ sample.

*Results*

Samples coming from reactions using the Pho-Zr-Pho trilayered catalyst supported onto polarized cHAp and an reducing atmosphere constituted by $N_2$, $CO_2$, $CH_4$ and $H_2O$ (set 1 in Table 4) gave rise to positive ninhydrin tests, suggesting therefore the formation of primary amines. In fact, purple spots were developed inside the recovered solids after reaction, indicating that amine compounds were mainly absorbed into the solid substrate. These compounds were well dissolved in the acetone solution after vigorous stirring, contrasting with the uncolored solid/solutions observed for other assayed reaction conditions (e.g. set 2 and sets 4 to 13 in Table 4).

**Table 4.** Summary of experiments and results attained for the synthesis of amino acids (AAs).[a]

| Set | Conditions[a] | Ninhidrine test | Observations |
|---|---|---|---|
| 1 | p-cHAp/Pho/Zr/Pho $N_2$, $CH_4$, $CO_2$, $H_2O$ | + | Gly and Ala signals in NMR spectra. Increasing AAs/Phos ratio with reaction time. Increasing AAs/Phos ratio with reaction *T*. Increasing AA/Phos ratio with Zr content. |
| 2 | p-cHAp/Phos/Zr/Phos $N_2$, $CH_4$, $CO_2$, $H_2O$ | - | UV radiation is fundamental. |
| 3 | p-aHAp/Pho/Zr/Pho $N_2$, $CH_4$, $CO_2$, $H_2O$ | + | The crystalline structure of HAp is not fundamental for reaction. |
| 4 | cHAp/Phos/Zr/Phos $N_2$, $CH_4$, $CO_2$, $H_2O$ | - | Polarization of HAp is fundamental. |
| 5 | p-N757/Phos/Zr/Phos $N_2$, $CH_4$, $CO_2$, $H_2O$ | - | The type of polarized support is important. |

(continued)

| Set | Conditions[a] | Ninhidrine test | Observations |
|---|---|---|---|
| 6 | p-LM/Phos/Zr/Phos $N_2$, $CH_4$, $CO_2$, $H_2O$ | - | The type of polarized support is important. |
| 7 | p-cHAp/Phos/Zr $N_2$, $CH_4$, $CO_2$, $H_2O$ | - | The trilayered system is fundamental. |
| 8 | p-cHAp/Zr/Phos $N_2$, $CH_4$, $CO_2$, $H_2O$ | - | The trilayered system is fundamental. |
| 9 | p-cHAp/Phos $N_2$, $CH_4$, $CO_2$, $H_2O$ | - | The trilayered system is fundamental. |
| 10 | p-cHAp/Zr $N_2$, $CH_4$, $CO_2$, $H_2O$ | - | The trilayered system is fundamental. |
| 11 | Phos | - | AAs cannot be derived from a simple decomposition of Phos using zirconate as catalyst. |
| 12 | Phos/Zr $N_2$, $CH_4$, $CO_2$, $H_2O$ | - | AAs cannot be derived from a simple decomposition of Phos. |
| 13 | p-cHAp/Phos/Zr/Phos $CH_4$, $CO_2$, $H_2O$ | - | Substrate is able to fix molecular nitrogen. Molecular nitrogen is imprescindible. |
| 14 | p-aHAp/Phos/Zr/Phos $N_2$, $CO_2$, $H_2O$ | - | $CH_4$ appears as the carbon source for $CH_2$ and $CH_3$ groups. |
| 15 | p-cHAp/Phos/Zr/Phos $N_2$, $CH_4$, $H_2O$ | - | $CO_2$ appears as the source for carboxylic groups. |
| 16 | p-cHAp/Phos/Zr $N_2$, $CH_4$, $CO_2$ | - | $H_2O$ play an important role in the mechanism. |

[a]Abbreviations denote the support (p-aHAp, aHAp, p-N757, p-LM) and the order of the different layers deposited onto its surface (Phos and Zr for phosphonate and zirconite, respectively). All experiments were performed under UV radiation except set 2.

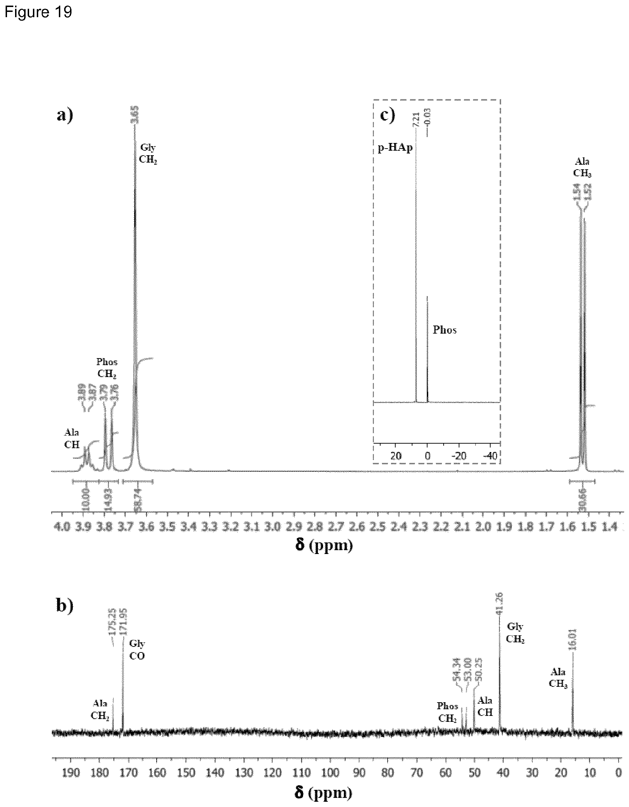

$^1$H NMR spectra (Figure 19a) showed only the presence after reaction of the phosphonate methylene group (i.e. doublet at 3.79-3.76 ppm) and signals corresponding to methylene protons of glycine (singlet at 3.65 ppm) and both methine (quadruplet at 3.91-3.85 ppm) and methane (doublet at 1.54-1.52 ppm) groups of alanine. The same compounds were also evidenced in the $^{13}$C NMR spectrum (Figure 19b) where only peaks assigned to the phosphonate (54.34 and 53.00 ppm), glycine (171.95 and 41.26 ppm) and alanine (175.25, 50.25 and 16.01 ppm) units could be detected. It is noteworthy that no by-products were observed and consequently a very clean process for production of glycine and alanine was developed.

$^1$H NMR spectra were analyzed for samples recovered after different reaction times (i.e. from 2 to 96 h), being possible to detect the ratios between glycine and phosphonate units (Gly/Phos), alanine and phosphonate units (Ala/Phos) and obviously between glycine and alanine unis (Gly/Ala). Specifically, the areas of signals corresponding to $CH_2$ protons at 3.65 and 3.79-3.76 ppm and the $CH_3$ protons at 1.54-1.52 ppm were considered:

$$Gly/Phos = (3 \times A_{3.65}) / A_{3.79\text{-}376} \qquad (1)$$

$$Ala/Phos = (2 \times A_{1.54\text{-}1.52}) / A_{3.79\text{-}376} \qquad (2)$$

$$Gly/Ala = (1.5 \times A_{3.65}) / A_{1.54\text{-}1.52} \qquad (3)$$

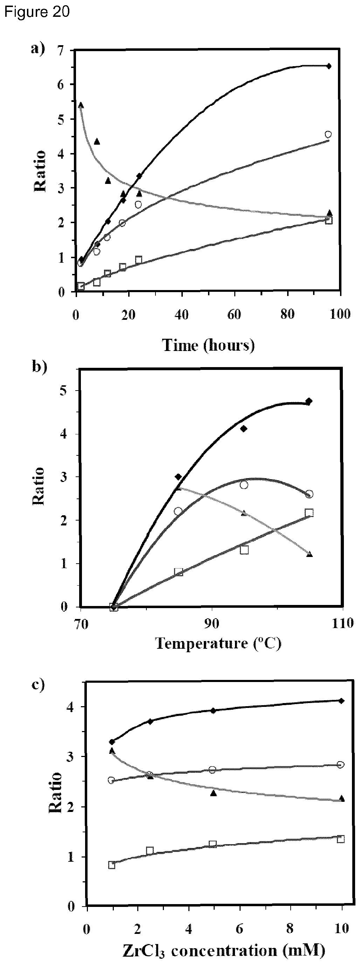

Results plotted in Figure 20a allow deducing that glycine is firstly produced and alanine is subsequently derived from this simple amino acid. Thus, the Gly/Ala ratio decreases from 5.4 to 2.2, being nevertheless observed a continuous increase of the Gly/Phos ratio with the reaction time (i.e. from 0.8 to 4.5).

Figure 20b allows estimating the effect of reaction temperature and specifically that a minimum value (i.e. 75 °C) is required to get a detectable amount of amino acids after 24 h of reaction. The Ala/Phos ratio continuosly increased with reaction temperature while the Gly/Phos ratio started to decrease at the maximum assayed temperature (105 °C) as a result of conversion of glycine into alanine. Nevertheless, the ratio between the total amino acid content and the phosphonate content still increased at this temperature.

Figure 20c shows as the content of the zirconate has a practically negligible influence on the Gly/Phos and Ala/Phos ratios, as presumable for a catalyst. Nevertheless, samples prepared from solutions with a very low concentration of zirconyl thrichloride (1 mM) led to significantly lower ratios as a consequence of the defective trilayered system. Logically, alanine was in this case the amino acid more disfavoured (i.e. the Gly/Ala ratio was maximum).

Experiments have also been assayed without exposure to the UV radiation (set 2), being in this case unsuccessful the formation of amino acids. Thus, the sustained exposure to UV logically appears as a fundamental issue to get radicals (e.g. $CH_3\cdot$) for further reaction towards formation of alanine and even glycine.

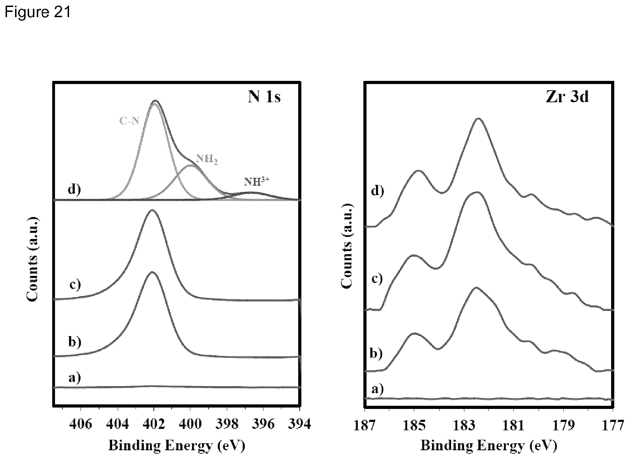

XPS analysis was fundamental to corroborate that amino acids were derived from the molecular nitrogen and not from a hypothetical decomposition of the phosphonate compound. Note that this point cannot be inferred from the NMR spectra since the increase of Gly/Phos could also be related to a decomposition process. Figure 21a shows the XPS spectra in the N1s region for different representative samples and specifically as a peak around 399 eV appears when phosphonate is incorporated onto the surface of p-cHAp. This peak is associated to the nitrogen in the C-N bond and is observed with practically the same intensity when both negative and positive reactions took place. Only in the last case additional peaks corresponding to the deprotonated ($NH_2$) and protonated ($NH_3^+$) amino groups were observed at 400.3 eV and 403.8.4 eV, respectively.[32] The amount of nitrogen increased from 0% to 2.75-2.97% when the Phos-Zr-Phos trilayer was deposited onto the p-cHAP substrate and to 6.2% after positive reaction (i.e. set 1 for 24 h at 95 °C). XPS spectra allowed determining the decrease of Ca/P ratio from a typical value of 1.64 for HAp to 1.26-1.29 when the trilayer was deposited onto the HAp surface. XPS spectra showed also Zr signals (Figure 21b) which appeared as a resolved spin doublet at binding energies of 182.6 (3d5/2) and 185 eV (3d3/2). The measured Zr content was in the 1.26-1.29% range for all samples having the Phos-Zr-Phos trilayer prepared from a 5 mM zirconyl chloride solution, being this percentage independently of the progress of the reaction.

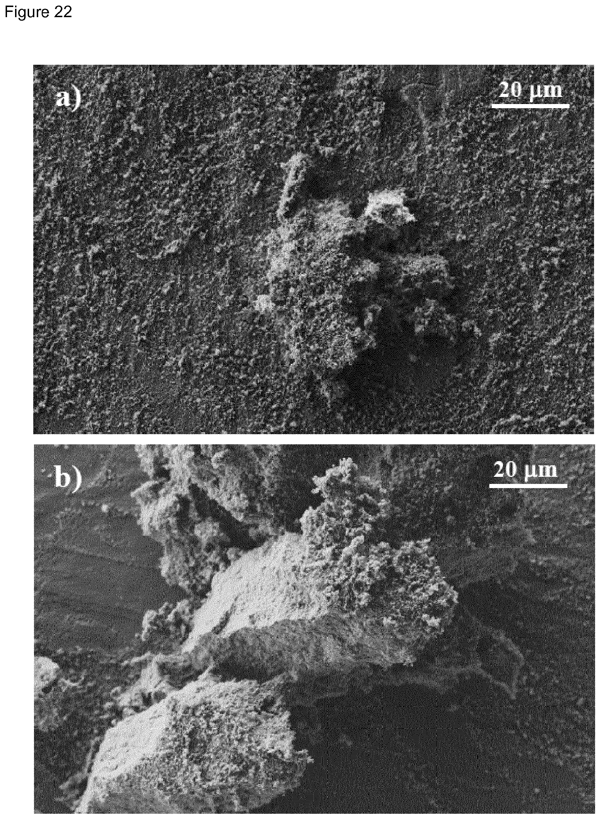

Deposition of the trilayered system on HAp gave rise to a rough and relative irregular disk surface as shown in the SEM micrograph corresponding to a polarized sample (Figure 22a). This surface slightly changed after reaction since a sporadic formation of regular crystals was detected. Figure 22b shows the growth of micrometric prismatic structures where the hexagonal basal plane tended to be parallel to the disk surface. In fact, it has been reported the capacity of organophosphonate films for inducing crystallization and grown of oriented molecular sieves. In this way, stable, vertically oriented and one dimensional aluminium phosphate crystals were able to grow over the hybrid layers. Cannel systems that could be applied as new catalytic membranes with true molecular selectivity and even for controlling the access of determined size to a sensor surface were formed. In any case, the present results demonstrate that amino acid crystals can also grow onto the surface of the trilayered catalyst but it should also be taken into account that the nynhydrin test revealed the presence of absorbed amino acids inside the disk sample.

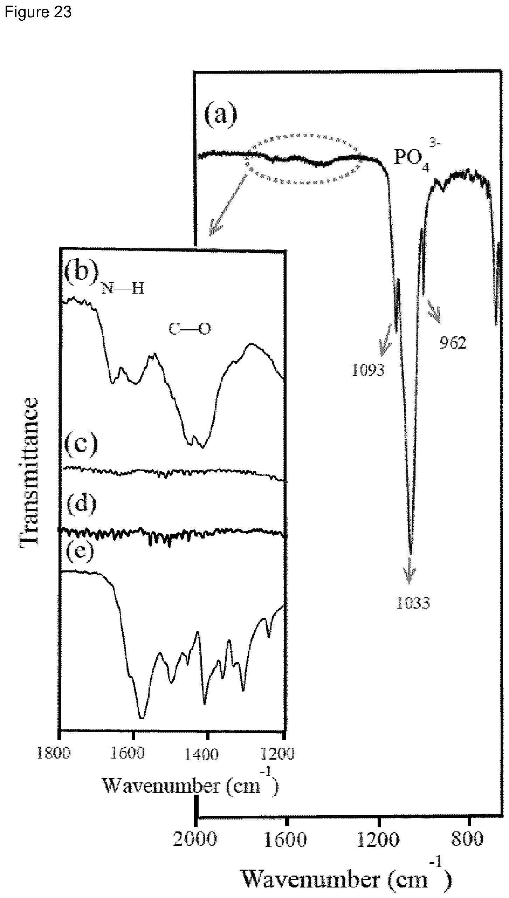

Significant differences between set 1 samples before and after reaction can be observed in the FTIR spectra despite the low sensitivity of the technique. Thus, broad and low-intensity bands in the 1600-1400 cm[-1] region could only be observed in the second case (Figure 23). It is worth noting that this region is completely flat in the spectra of samples before reaction and also for samples coming from a negative ninhydrin test (e.g. set 2 samples). On the contrary, amino acids such as glycine and alanine have the most intense absorptions in this region (see inset of Figure 23). Logically, FTIR spectra showed the characteristic peaks of HAp and specifically the three intense bands at 1093, 1033 and 962 cm-[1] associated to the characteristic vibrational modes of $PO_4^{3-}$ were always observed.

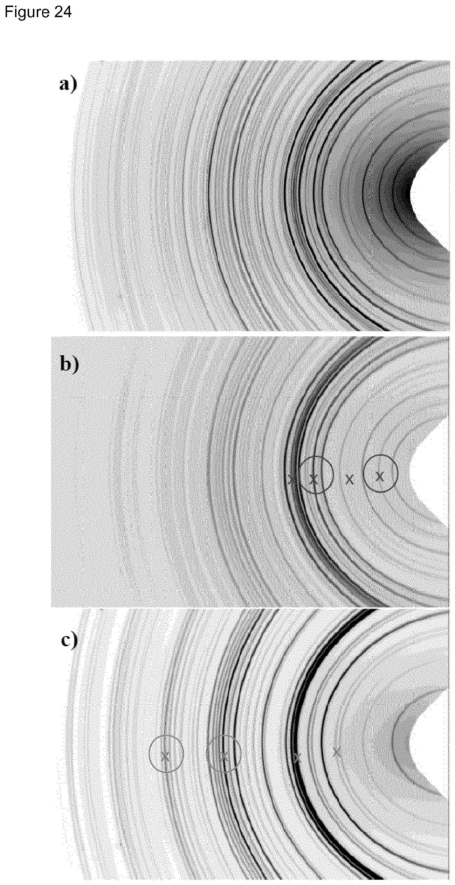

Deposition of the trilayered systems over the polarized c-HAp did not cause a significant change on the X-ray diffraction pattern (Figure 24a and 24b) whereas remarkable changes can be observed after chemical reaction (Figures 24b and 24c).

### *Influence of changes on the catalyst system and the polarized support on the synthesis of hydroxyapatite*

Amino acids were also detected when p-aHAp was employed instead of p-cHAp and the experimental conditions of set 1 were maintained. Nevertheless, we preferred to insist on p-cHAp since the amorphous sample suffered a partial decomposition during the sintering process, which led to the formation of β-tricalcium phosphate (β-TCP: $\beta\text{-}Ca_3(PO_4)_2$) as the predominant phase.

Different assays have been performed in order to evaluate the importance of the type of substrate of the catalytic system. Amino acids were only detected when polarized HAp was employed (e.g. sets 1 and 3 in Table 4), being highly significant the negative results obtained when sintered HAp (set 4) was used as a substrate and also when other systems such as silicates (e.g. Nanofil 757, set 5) and aluminosilicates (e.g. layered mica, set 5) were tested even after being polarized under similar conditions to those applied for p-cHAp.

The suitability of the HAp contribution is interesting since it plays a fundamental role in living systems and specifically constitutes their most abundant inorganic component. The relationship between HAp and biological molecules (e.g. proteins like collagen and even DNA constituted by a phosphate skeleton) has nowadays enhanced an intensive research on its use for different biomedical applications (e.g. drug and gene delivery, bone repair and tissue engineering among others).

In this sense, it is also remarkable that the metal/phosphonate layered system is also able to molecular recognition and consequently a selective binding of an enantiomeric compound from a racemic solution can be achieved. Furthermore, the high insolubility and stability towards thermal treatments and chemical reactants of zirconium phosphonates have opened other potential applications such as viral vectors in gene delivery. The positive charge of amino-functionalized phosphonates (e. g. the aminoethoxy derivative) allows the direct intercalation of negatively charged DNA molecules. Moreover, binding is pH sensitive being found that the conformation of DNA could be almost retained during intercalation and release processes.

For the sake of completeness we have also assayed the effectiveness of the two possible bilayered (deposition of a first layer of Phos or Zr and subsequent deposition of the second complementary layer, sets 7 and 8, respectively) and monolayer (sets 9 and 10) systems. In all cases, negative results were attained demonstrating that a stable Phos-Zr complex with a nucleation activity was only attained using the trilayered architecture. Probably dissolution of components in the water reaction medium should also be taken into account when bilayered and monolayer arrangements are considered.

Table 4 reports also the results attained when only phosphonate (set 11) and even a mixture of phosphonate and zirconyl chloride (set 12) were introduced into the reactor instead of the coated polarized support. These assays are also relevant since help discarding a process based on the decomposition of AMTP. Note that in this case the amount of AMTP submitted to UV irradiation and able to react with the selected reducing atmosphere was much higher than required in the trilayered system.

## REFERENCES

1. S. V. Dorozhkin and M. Epple, Angew. Chem., Int. Ed., 2002, 41, 3130.

2. L. C. Palmer, C. J. Newcomb, S. R. Kaltz, E. D. Spoerke and S. I. Stupp, Chem. Rev., 2008, 108, 4754.

3. M. Y.Ma, Y. J. Zhu, L. Li and S. W. Cao, J. Mater. Chem., 2008, 18, 2722.

4. K. W. Wang, L. Z. Zhou, Y. Sun, G. J. Wu, H. C. Gu, Y. R. Duan, F. Chen and Y. J. Zhu, J. Mater. Chem., 2010, 20, 1161.

5. Q. L. Tang, Y. J. Zhu, J. Wu, F. Chen and S. W. Cao, Nanomed.: Nanotechnol., Biol. Med., 2011, 7, 428.

6. H.-W. Kim, J. C. Knowles, and H.-E. Kim, Biomaterials 25, 1279 (2004).

7. W. Suchanek and M. Yoshimura, J. Mater. Res., 1998, 13, 94.

8. H. Zhou and J. Lee, Acta Biomater., 2011, 7, 2769.

9. J. C. Elliott, P. E. Mackie, and R. A. Young, Science 180, 1055 (1973).

10. G. Ma and X. Y. Liu, Cryst. Growth Des. 9, 2991 (2009).

11. N. Hitmi, C. LaCabanne, and R. A. Young, J. Phys. Chem. Solids 49, 541 (1988).

12. T. Ikoma, A. Yamazaki, S. Nakamura, and M. Akao, J. Mater. Sci. Lett. 18, 1225 (1999).

13. I. M. Kalogeras, A. Vassilikou-Dova, and A. Katerinopoulou, J. Appl. Phys. 92, 406 (2002).

14. N. Horiuchi, M. Nakamura, A. Nagai, K. Katayama and K. Yamashita, J. Appl. Phys., 2012, 112, 074901.

15. N. Horiuchi, S. Nakaguki, N. Wada, M. Nakamura, A. Nagai, K. Katayama and K. Yamashita, J. Appl. Phys., 2014, 116, 014902.

16. Nakamura, M.; Hori, N.; Namba, S.; Toyama, T.; Nishimiya, N.; Yamashita, K. Biomed. Mater. 2015, 10, 011001.

17. M. Nakamura, A. Nagai, T. Hentunen, J. Salonen, Y. Sekilima, T. Okura, K. Hashimoto, Y. Toda, H. Monma, K. Yamashita, ACS Appl. Mater. Interfaces, 2009, 1, 2182.

18. M. Rivas, J. Casanovas, L. J. del Valle, O. Bertran, G. Revilla-López, P. Turón, J. Puiggalí, C. Alemán Dalton Trans., 2015, 44, 9980-9991.

19. K. D. Kumble and A. Kornberg, J. Biol. Chem., 1996, 270, 5818-5822.

20. K. Doi, T. Kubi, R. Takeshita, S. Kajihara, S. Kato, Y. Kawazoe, T. Shiba and Y. Akagawa, Dent. Mat. J., 2014, 33, 179-186.

21. P.A. Comeau, H. Frei, C.Yang, G. Fernlund and F.M. Rossi, J. Biomat. Appl., 2012, 27, 267-275.

22. K. Siggers, H. Frei, G. Fernlund, and F. Rossi, J. Biomed. Mat. Res. Part A, 2010, 94, 877-885.

23. K. Morita, K. Doi, T. Kubo, R. Takeshita, S. Kato and Y. Akagawa, Acta Biomat., 2010, 6,2808-2815.

24. Q. Yuan, T. Kubo, K. Doi, K. Morita, R. Takeshita, S. Kato, T. Shiba and Y. Akagawa, Acta Biomat., 2009, 5, 1716-1724.

25. T. Shiba, D. Nishimura, Y. Kawazoe, Y. Onodera, K. Tsutsumi, R. Nakamura and M. Ohshiro, J. Biol. Chem., 2003, 278, 26788-26792.

26. Y. Kawazoe, T. Shiba, R. Nakamura, A. Mizuno, K. Tsutsumi, T. Uematsu, M. Yamaoka, M. Shindoh and T. Kohgo, J. Dent. Res., 2004, 83, 613-618.

27. Y. Hacchou, T. Uematsu, O. Ueda, Y. Usui, S. Uematsu, M. Takahashi, Y. Kawazoe, T. Shiba, S. Kurihara, M. Yamaoka and K. Furusawa, J. Dent. Res., 2007, 86, 893-897.

28. H. Fleisch and S. Bisaz, Nature, 1962, 195, 911-911.

29. H. Fleish, R. Russel and F. Straumann, Nature, 1966, 212, 901-903.

30. S. Omelon, J. Georgiou, Z. J. Henneman, L. M. Wise, B. Sukhu, T. Hunt, C. Wynnyckyj, D. Holmyard, R. Bielecki, and M. D. Grynpas, PLos One, 2009, 4, e5634.

31. S. S. Kamat and F. M. Raushel, Curr. Opin. Chem. Bio., 2013, 17, 589-596.

32. F. H. Ebetino and R. G. G. Russell, J. Bone Miner Res., 2005, 20, 259.

33. R. G. G. Russell and F.H. Ebetino, Osteoporos. Int., 2008, 19, 733-759.

34. N. Gronich and G. Rennet, Nat. Rev. Clin. Oncol., 2013, 10, 625-642.

35. L. J. del Valle, O. Bertran, G. Chaves, G. Revilla-López, M. Rivas, M. T. Casas, J. Casanovas, P. Turon, J. Puiggalí, C. Alemán J. Mater. Chem. B, 2014, 2, 6953-6966.

36. H. Klug and L. Alexander in X-Ray Diffraction Procedure for Polycrystallite and Amorphous Materials, 2nd. Edition, John Wiley and Sons, New York, 1974).

37. E. Landi, A. Tampieri, G. Celotti and S. Sprio, J. Eur. Ceram. Soc., 2000, 20, 2377-2387.

38. F. Estrany, D. Aradilla, R. Oliver and C. Alemán, Eur. Polym. J., 2007, 43, 1876.

39. F. Müller, C. A. Ferreira, D. S. Azambuja, C. Alemán, E. Armelin, Measuring the Proton Conductivity of Ion-Exchange Membranes Using Electrochemical Impedance Spectroscopy and Through-Plane Cell, J. Phys. Chem. B 2014, 118, 1102-1112.

40. S. Raynaud, E. Champion, Bernache-Assollant, P. Thomas. Calcium phosphate apatites with variable Ca/P atomic ratio I: synthesis, characterization and thermal stability of powders. Biomaterials, 23, 1065-1072 (2002).

41. H. Fujimori, H. Toya, K. Ioku, S. Goto, and M. Yoshimura, Chem. Phys. Lett. 325, 383 (2000).

42. J. C. Elliott, P. E. Mackie, and R. A. Young, Science 180, 1055 (1973).

43. N. Hitmi, C. LaCabanne, and R. A. Young, J. Phys. Chem. Solids 49, 541 (1988).

44. G. Ma and X. Y. Liu, Cryst. Growth Des. 9, 2991 (2009).

45. Handbook of X-ray Photoelectron Spectroscopy (Eds.: J. F. Moulder, J. Chastain), Physical Electronics Division, PerkinElmer Corporation, 1995.

46. M. C. Chang, J. Tanaka, Biomaterials 2002, 23, 3879- 3885.

47. Bertran, O.; del Valle, L. J.; Revilla-López, G.; Rivas, M.; Chaves, G.; Casas, M. T.; Casanovas, J.; Turon, P.; Puiggalí, J. Chem. Eur. J. 2015, 21,2537-2546.

48. I. Ming-Hung, W.-J. Shih, M.-H. Hon, M.-C. Wang, Int. J. Mol. Sci. 2012, 13, 13569-13586.

49. J.P. Gittings, C.R. Bowen, A.C.E. Dent, I.G. Turner, F.R. Baxter, J.B. Chaudhuri, Electrical characterization of hydroxyapatite-based bioceramics. Acta Biomaterialia 5 (2009) 743-754.

50. M. J. Lukic, C. Jovalekic, S. Markovic, D. Uskolovic. Enhanced high-temperature electrical response of hydroxyapatite upon grain size refinement. Materials Research Bulletin 61 534-538 (2014).

51. Y. Liu, Z. Shen. Dehydroxylation of hydroxyapatite in dense bulk ceramics sintered by spark plasma sintering. J. Eur. Ceram. Soc. 32 (11), 2691-2696 (2012).

52. S. Tarafder, S. Banerjee, A. Bandyopadhyay, S. Bose. Langmuir 2010, 26, 16625-16629.

53. K.J. Gaskell, A.L. Asunkis, P.M.A. Sherwood. Sodium Pyrophosphate Decahydrate (Na4P2O7·10H2O) byXPS. Surface Science Spectra, vol 9, 135-142(2004)

54. K.J. Gaskell, A.L. Asunkis, P.M.A. Sherwood. Sodium Tripolyphosphate (Na5P3O10) by XPS. Surface Science Spectra, vol 9, 166-173 (2004).

55. V. Dalmoro, J. H. Z. dos Santos, E. Armelin, C. Alemán and D. Azambuja, Appl. Surf. Sci., 2013, 273, 758-768.

## Claims

1. Process for obtaining a permanently polarized hydroxyapatite comprising the steps of:

   (a) obtaining sintered samples of crystalline hydroxyapatite and amorphous calcium phosphate;
   (b) heating the samples obtained in (a) at a temperature between at least 1000°C and 1200°C;
   (c) applying a constant DC voltage between 250 and 2500 V for at least 1 minute or an electrostatic discharge between 2500 and 1500000 V for less than 10 minutes;

(d) cooling the samples maintaining the DC voltage.

2. Process, according to claim 1, wherein the step (a) is carried out at a temperature between 800 and 1100°C.

3. Process according to claim 1 or 2, wherein the constant DC voltage in step (c) is applied for 0.5 to 1.5 hours.

**Patentansprüche**

1. Verfahren zur Herstellung von permanent polarisiertem Hydroxyapatit, umfassend die Stufen:

(a) Gewinnen von gesinterten Proben von kristallinem Hydroxyapatit und amorphem Calciumphosphat;
(b) Erwärmen der in (a) erhaltenen Proben auf eine Temperatur zwischen mindestens 1000 °C und 1200 °C;
(c) Anlegen einer konstanten Gleichstromspannung zwischen 250 und 2500 V für mindestens 1 Minute oder einer elektrostatischen Entladung zwischen 2500 und 1500000 V für weniger als 10 Minuten;
(d) Abkühlen der Proben unter Aufrechterhaltung der Gleichstromspannung.

2. Verfahren nach Anspruch 1, wobei die Stufe (a) bei einer Temperatur zwischen 800 und 1100 °C durchgeführt wird.

3. Verfahren nach Anspruch 1 oder 2, wobei die konstante Gleichstromspannung in Stufe (c) für 0,5 bis 1,5 Stunden angelegt wird.

**Revendications**

1. Procédé d'obtention d'une hydroxyapatite à polarisation permanente comprenant les étapes consistant à :

(a) obtenir des échantillons frittés d'hydroxyapatite cristalline et de phosphate de calcium amorphe ;
(b) chauffer les échantillons obtenus en (a) à une température entre au moins 1 000 °C et 1 200 °C ;
(c) appliquer une tension CC constante entre 250 et 2 500 V pendant au moins 1 minute ou une décharge électrostatique entre 2 500 et 1 500 000 V pendant moins de 10 minutes ;
(d) refroidir les échantillons en maintenant la tension CC.

2. Procédé, selon la revendication 1, dans lequel l'étape (a) est effectuée à une température entre 800 et 1 100 °C.

3. Procédé selon la revendication 1 ou 2, dans lequel la tension CC constante à l'étape (c) est appliquée pendant 0,5 à 1,5 heure.

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

Figure 16

Figure 17

Figure 18

A)

31P{1H} - HPDEC.AV, 400 MHz
referenced to H3PO4 (85%)
sample name: C1
sample spinning rate: 12 KHz
sample owner: Carlos Aleman, UPC
recovery delay d1: 5s
number of scans: 256
overall experimental time: 20 min
hard 31P 90° pulse: 5 us (19 w)
cpd-1H: spinal64 (73 w)
processing: FT with exponential function (lb 5 Hz)

B)

31P{1H} - HPDEC.AV, 400 MHz
referenced to H3PO4 (85%)
sample name: C2
sample spinning rate: 12 KHz
sample owner: Carlos Aleman, UPC
recovery delay d1: 5s
number of scans: 256
overall experimental time: 20 min
hard 31P 90° pulse: 5 us (19 w)
cpd-1H: spinal64 (73 w)
processing: FT with exponential function (lb 5 Hz)

C)

```
31P{1H} - HPDEC.AV, 400 MHz
referenced to H3PO4 (85%)
sample name: C3
sample spinning rate: 12 KHz
sample owner: Carlos Aleman, UPC
recovery delay d1: 5s
number of scans: 256
overall experimental time: 20 min
hard 31P 90° pulse: 5 us (19 w)
cpd-1H: spinal64 (73 w)
processing: FT with exponential function (lb 5 Hz)
```

D)

Figure 19

Figure 20

Figure 21

Figure 22

Figure 23

Figure 24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- ES 2323628

### Non-patent literature cited in the description

- **FU, CONG et al.** Hydroxyapatite thin films with giant electrical polarization. *chemistry of Materials,* 2015, vol. 27 (4), 1164-1171
- Electrovectorial effect of polarized hydroxyapatite on quasiepitaxial growth at nano-interfaces. **UESHIMA M et al.** Solid State Ionics. North Holland Pub. Company, 01 November 2002, vol. 151, 29-34
- **MIHO NAKAMURA et al.** Role of blood coagulation components as intermediators of high osteoconductivity of electrically polarized hydroxyapatite. *Journal of biomedical Material Research,* 01 December 2006, vol. 79 (3), 627-634
- Local structure of hydroxy-peroxy apatite: A combined XRD, FT-IR, Raman, SEM, and solid-state NMR study. **YU et al.** Journal of Physics and Chemistry of Solids. Pergamon Press, 01 October 2007, vol. 68, 1863-1871
- **S. V. DOROZHKIN ; M. EPPLE.** *Angew. Chem., Int. Ed.,* 2002, vol. 41, 3130
- **L. C. PALMER ; C. J. NEWCOMB ; S. R. KALTZ ; E. D. SPOERKE ; S. I. STUPP.** *Chem. Rev.,* 2008, vol. 108, 4754
- **M. Y.MA ; Y. J. ZHU ; L. LI ; S. W. CAO.** *J. Mater. Chem.,* 2008, vol. 18, 2722
- **K. W. WANG ; L. Z. ZHOU ; Y. SUN ; G. J. WU ; H. C. GU ; Y. R. DUAN ; F. CHEN ; Y. J. ZHU.** *J. Mater. Chem.,* 2010, vol. 20, 1161
- **Q. L. TANG ; Y. J. ZHU ; J. WU ; F. CHEN ; S. W. CAO.** *Nanomed.: Nanotechnol., Biol. Med.,* 2011, vol. 7, 428
- **H.-W. KIM ; J. C. KNOWLES ; H.-E. KIM.** *Biomaterials,* 2004, vol. 25, 1279
- **W. SUCHANEK ; M. YOSHIMURA.** *J. Mater. Res.,* 1998, vol. 13, 94
- **H. ZHOU ; J. LEE.** *Acta Biomater.,* 2011, vol. 7, 2769
- **J. C. ELLIOTT ; P. E. MACKIE ; R. A. YOUNG.** *Science,* 1973, vol. 180, 1055
- **G. MA ; X. Y. LIU.** *Cryst. Growth Des.,* 2009, vol. 9, 2991
- **N. HITMI ; C. LACABANNE ; R. A. YOUNG.** *J. Phys. Chem. Solids,* 1988, vol. 49, 541
- **T. IKOMA ; A. YAMAZAKI ; S. NAKAMURA ; M. AKAO.** *J. Mater. Sci. Lett.,* 1999, vol. 18, 1225
- **I. M. KALOGERAS ; A. VASSILIKOU-DOVA ; A. KATERINOPOULOU.** *J. Appl. Phys.,* 2002, vol. 92, 406
- **N. HORIUCHI ; M. NAKAMURA ; A. NAGAI ; K. KATAYAMA ; K. YAMASHITA.** *J. Appl. Phys,* 2012, vol. 112, 074901
- **N. HORIUCHI ; S. NAKAGUKI ; N. WADA ; M. NAKAMURA ; A. NAGAI ; K. KATAYAMA ; K. YAMASHITA.** *J. Appl. Phys.,* 2014, vol. 116, 014902
- **NAKAMURA, M. ; HORI, N. ; NAMBA, S. ; TOYAMA, T. ; NISHIMIYA, N. ; YAMASHITA, K.** *Biomed. Mater.,* 2015, vol. 10, 011001
- **M. NAKAMURA ; A. NAGAI ; T. HENTUNEN ; J. SALONEN ; Y. SEKILIMA ; T. OKURA ; K. HASHIMOTO ; Y. TODA ; H. MONMA ; K. YAMASHITA.** *ACS Appl. Mater. Interfaces,* 2009, vol. 1, 2182
- **M. RIVAS ; J. CASANOVAS ; L. J. DEL VALLE ; O. BERTRAN ; G. REVILLA-LÓPEZ ; P. TURÓN ; J. PUIGGALÍ ; C. ALEMÁN.** *Dalton Trans.,* 2015, vol. 44, 9980-9991
- **K. D. KUMBLE ; A. KORNBERG.** *J. Biol. Chem.,* 1996, vol. 270, 5818-5822
- **K. DOI ; T. KUBI ; R. TAKESHITA ; S. KAJIHARA ; S. KATO ; Y. KAWAZOE ; T. SHIBA ; Y. AKAGAWA.** *Dent. Mat. J.,* 2014, vol. 33, 179-186
- **P.A. COMEAU ; H. FREI ; C.YANG ; G. FERNLUND ; F.M. ROSSI.** *J. Biomat. Appl.,* 2012, vol. 27, 267-275
- **K. SIGGERS ; H. FREI ; G. FERNLUND ; F. ROSSI.** *J. Biomed. Mat. Res.,* 2010, vol. 94, 877-885
- **K. MORITA ; K. DOI ; T. KUBO ; R. TAKESHITA ; S. KATO ; Y. AKAGAWA.** *Acta Biomat.,* 2010, vol. 6, 2808-2815
- **Q. YUAN ; T. KUBO ; K. DOI ; K. MORITA ; R. TAKESHITA ; S. KATO ; T. SHIBA ; Y. AKAGAWA.** *Acta Biomat.,* 2009, vol. 5, 1716-1724
- **T. SHIBA ; D. NISHIMURA ; Y. KAWAZOE ; Y. ONODERA ; K. TSUTSUMI ; R. NAKAMURA ; M. OHSHIRO.** *J. Biol. Chem.,* 2003, vol. 278, 26788-26792

- **Y. KAWAZOE ; T. SHIBA ; R. NAKAMURA ; A. MIZUNO ; K. TSUTSUMI ; T. UEMATSU ; M. YAMAOKA ; M. SHINDOH ; T. KOHGO.** *J. Dent. Res.,* 2004, vol. 83, 613-618
- **Y. HACCHOU ; T. UEMATSU ; O. UEDA ; Y. USUI ; S. UEMATSU ; M. TAKAHASHI ; Y. KAWAZOE ; T. SHIBA ; S. KURIHARA ; M. YAMAOKA.** *J. Dent. Res.,* 2007, vol. 86, 893-897
- **H. FLEISCH ; S. BISAZ.** *Nature,* 1962, vol. 195, 911-911
- **H. FLEISH ; R. RUSSEL ; F. STRAUMANN.** *Nature,* 1966, vol. 212, 901-903
- **S. OMELON ; J. GEORGIOU ; Z. J. HENNEMAN ; L. M. WISE ; B. SUKHU ; T. HUNT ; C. WYNNYCKYJ ; D. HOLMYARD ; R. BIELECKI ; M. D. GRYNPAS.** *PLos One,* 2009, vol. 4, e5634
- **S. S. KAMAT ; F. M. RAUSHEL.** *Curr. Opin. Chem. Bio.,* 2013, vol. 17, 589-596
- **F. H. EBETINO ; R. G. G. RUSSELL.** *J. Bone Miner Res.,* 2005, vol. 20, 259
- **R. G. G. RUSSELL ; F.H. EBETINO.** *Osteoporos. Int.,* 2008, vol. 19, 733-759
- **N. GRONICH ; G. RENNET.** *Nat. Rev. Clin. Oncol.,* 2013, vol. 10, 625-642
- **L. J. DEL VALLE ; O. BERTRAN ; G. CHAVES ; G. REVILLA-LÓPEZ ; M. RIVAS ; M. T. CASAS ; J. CASANOVAS ; P. TURON ; J. PUIGGALÍ ; C. ALEMÁN.** *J. Mater. Chem. B,* 2014, vol. 2, 6953-6966
- **H. KLUG ; L. ALEXANDER.** X-Ray Diffraction Procedure for Polycrystallite and Amorphous Materials. John Wiley and Sons, 1974
- **E. LANDI ; A. TAMPIERI ; G. CELOTTI ; S. SPRIO.** *J. Eur. Ceram. Soc.,* 2000, vol. 20, 2377-2387
- **F. ESTRANY ; D. ARADILLA ; R. OLIVER ; C. ALEMÁN.** *Eur. Polym. J.,* 2007, vol. 43, 1876
- **F. MÜLLER ; C. A. FERREIRA ; D. S. AZAMBUJA ; C. ALEMÁN ; E. ARMELIN.** Measuring the Proton Conductivity of Ion-Exchange Membranes Using Electrochemical Impedance Spectroscopy and Through-Plane Cell. *J. Phys. Chem. B,* 2014, vol. 118, 1102-1112
- **S. RAYNAUD ; E. CHAMPION ; BERNACHE-ASSOLLANT ; P. THOMAS.** Calcium phosphate apatites with variable Ca/P atomic ratio I: synthesis, characterization and thermal stability of powders. *Biomaterials,* 2002, vol. 23, 1065-1072
- **H. FUJIMORI ; H. TOYA ; K. LOKU ; S. GOTO ; M. YOSHIMURA.** *Chem. Phys. Lett.,* 2000, vol. 325, 383
- Physical Electronics Division. Handbook of X-ray Photoelectron Spectroscopy. PerkinElmer Corporation, 1995
- **M. C. CHANG ; J. TANAKA.** *Biomaterials,* 2002, vol. 23, 3879-3885
- **BERTRAN, O. ; DEL VALLE, L. J. ; REVILLA-LÓPEZ, G. ; RIVAS, M. ; CHAVES, G. ; CASAS, M. T. ; CASANOVAS, J. ; TURON, P. ; PUIGGALÍ, J.** *Chem. Eur. J.,* 2015, vol. 21, 2537-2546
- **I. MING-HUNG ; W.-J. SHIH ; M.-H. HON ; M.-C. WANG.** *Int. J. Mol. Sci.,* 2012, vol. 13, 13569-13586
- **J.P. GITTINGS ; C.R. BOWEN ; A.C.E. DENT ; I.G. TURNER ; F.R. BAXTER ; J.B. CHAUDHURI.** Electrical characterization of hydroxyapatite-based bioceramics. *Acta Biomaterialia,* 2009, vol. 5, 743-754
- **M. J. LUKIC ; C. JOVALEKIC ; S. MARKOVIC ; D. USKOLOVIC.** Enhanced high-temperature electrical response of hydroxyapatite upon grain size refinement. *Materials Research Bulletin,* 2014, vol. 61, 534-538
- **Y. LIU ; Z. SHEN.** Dehydroxylation of hydroxyapatite in dense bulk ceramics sintered by spark plasma sintering. *J. Eur. Ceram. Soc.,* 2012, vol. 32 (11), 2691-2696
- **S. TARAFDER ; S. BANERJEE ; A. BANDYOPADHYAY ; S. BOSE.** *Langmuir,* 2010, vol. 26, 16625-16629
- **K.J. GASKELL ; A.L. ASUNKIS ; P.M.A. SHERWOOD.** Sodium Pyrophosphate Decahydrate (Na4P2O7·10H2O) byXPS. *Surface Science Spectra,* 2004, vol. 9, 135-142
- **K.J. GASKELL ; A.L. ASUNKIS ; P.M.A. SHERWOOD.** Sodium Tripolyphosphate (Na5P3O10) by XPS. *Surface Science Spectra,* 2004, vol. 9, 166-173
- **V. DALMORO ; J. H. Z. DOS SANTOS ; E. ARMELIN ; C. ALEMÁN ; D. AZAMBUJA.** *Appl. Surf. Sci.,* 2013, vol. 273, 758-768